(19) Europäisches Patentamt
European Patent Office
Office européen des brevets

(11) **EP 2 680 769 B1**

(12) ## EUROPÄISCHE PATENTSCHRIFT

(45) Veröffentlichungstag und Bekanntmachung des Hinweises auf die Patenterteilung:
**27.04.2016 Patentblatt 2016/17**

(21) Anmeldenummer: **12701067.6**

(22) Anmeldetag: **25.01.2012**

(51) Int Cl.:
***A61B 17/68*** (2006.01)

(86) Internationale Anmeldenummer:
**PCT/EP2012/000329**

(87) Internationale Veröffentlichungsnummer:
**WO 2012/116772 (07.09.2012 Gazette 2012/36)**

(54) **SPANNELEMENT ZUM FIXIEREN EINER KNOCHENFRAKTUR SOWIE SELBIGES AUFWEISENDE MODULARE FIXIERUNGSVORRICHTUNG UND VERFAHREN ZUR HERSTELLUNG HIERFÜR**

CLAMPING ELEMENT FOR SETTING A BONE FRACTURE, MODULAR SETTING DEVICE COMPRISING SAME AND METHOD FOR PRODUCING SAME

ÉLÉMENT DE SERRAGE POUR LA FIXATION D'UNE FRACTURE OSSEUSE, DISPOSITIF DE FIXATION MODULAIRE POURVU DUDIT ÉLÉMENT ET PROCÉDÉ DE FABRICATION ASSOCIÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.03.2011 DE 102011001016**

(43) Veröffentlichungstag der Anmeldung:
**08.01.2014 Patentblatt 2014/02**

(73) Patentinhaber: **Hipp Medical AG**
**78600 Kolbingen (DE)**

(72) Erfinder: **WAIZENEGGER, Markus**
**78600 Kolbingen (DE)**

(74) Vertreter: **Kuhnen & Wacker**
**Patent- und Rechtsanwaltsbüro**
**Prinz-Ludwig-Straße 40A**
**85354 Freising (DE)**

(56) Entgegenhaltungen:
**WO-A1-2008/005740    WO-A1-2009/039430**
**WO-A2-2005/086708    WO-A2-2006/031692**
**WO-A2-2007/127628    US-A- 2 580 821**
**US-A1- 2009 163 954**

**Beschreibung**

Die Erfindung betrifft eine Fixierungsvorrichtung für Knochenfrakturen nach Anspruch 1 sowie mit Herstellungsverfahren hierfür nach den Ansprüchen 15 und 16.

Bei unzureichender Ruhigstellung einer Knochenfraktur kann eine Kallusbildung, d.h. eine schwielenartige Verdickung der Bruchenden aus überwachsendem Knochengewebe, auftreten. Um eine solche indirekte Frakturheilung über einen Kallus zu vermeiden, werden Knochenplatten verwendet, die auf der Außenfläche eines gebrochenen Knochens aufgebracht und befestigt werden, damit die Bruchstelle während des Heilungsprozesses fixiert ist.

Für derartige Anwendungen zur Versorgung von Knochenfrakturen sind aus dem Stand der Technik zumeist starre, flächig ausgebildete, metallische Knochenplatten mit Bohrungen bekannt, die an den gegenüberliegenden Seiten einer Fraktur verschraubt werden. Zur Befestigung solcher Fixierungen werden üblicherweise sogenannte Corticalisschrauben verwendet. Diese werden in das äußere Knochengewebe, die sogenannte Corticalisschicht, welche die höchste Festigkeit des Knochens aufweist, eingeschraubt. Die Fixierung stellt sicher, dass sich die Bruchenden nicht gegeneinander bewegen können und sich neugebildetes Knochengewebe belastungsfrei anlagern kann.

Zudem wird angenommen, dass der Heilungsprozess einer Fraktur günstig beeinflusst werden kann, wenn eine Kompression auf die zusammengefügte Bruchstelle aufgebracht wird. Hierdurch wird eine besonders enge Adaption, d.h. ein geringes Spaltmaß, über welches die Bruchenden wieder aufeinander zu wachsen, bewirkt.

Hierzu offenbart die US 7,763,056 B2 eine zweigeteilte Knochenplatte, deren Hälften sich mittels einer axialen Führung aufeinander zu bewegen können. Zur Herstellung einer Kompression werden verschiedene Anordnungen von Zugfedern vorgeschlagen, die an den beiden Hälften eingespannt sind. Die Knochenplatte ist aus mehreren Komponenten aufgebaut und dementsprechend kostenintensiv in der Herstellung.

Weiterhin sind vertebrale Knochenplatten bekannt, die nicht vollständig starr sind, sondern in einem geringen Umfang eine Annährung von Wirbelkörpern und ein Verschwenken bzw. Verdrehen der Wirbelkörper gegeneinander ermöglichen. Hierdurch kann z.B. die Regeneration einer geschädigten Bandscheibe oder das Ausheilen eines Wirbelkörperbruchs unterstützt werden. Die Gebrauchsmusterschrift DE 200 01 879 U1 und die Offenlegungsschrift WO 2004/034916 A1 offenbaren hierzu Knochenplatten mit meanderförmigen Abschnitten, die dem Aufbau der Knochenplatte eine multidirektionale Flexibilität verleihen.

Die Offenlegungsschrift WO 2005/086708 A2 beschreibt darüber hinaus verschiedene Ausführungsformen von Knochenplatten für die vordere Halswirbelsäule, die eine axiale und eine seitliche Flexibilität ermöglicht. Diese weisen einen Aufbau mit Zwischenabschnitten auf, die sich bei einer Belastung in axialer Richtung seitlich beugen.

Die Offenlegungsschrift WO2006031692 A2 offenbart eine Fixierungsvorrichtung gemäß dem Oberbegriff von Anspruch 1.

Derartige, aus dem Stand der Technik bekannte, vertebrale Knochenplatten sollen dabei jedoch eine Kompression zwischen zwei Wirbelkörpern verhindern, um die Bandscheibe zu entlasten und eine Beeinträchtigung der Spinalnerven zu vermeiden. Sie sind daher zur kompressionserzeugenden Fixierung einer Knochenfraktur ungeeignet und weisen darüber hinaus aufgrund der vielseitigen Anforderungen dieser besonders sensiblen Anwendung einen komplexen Aufbau auf. Auch die eingangs zitierten Knochenplatten sind relativ komplex gestaltet und mit einer entsprechend kostenintensiven Herstellung verbunden.

Somit wird bislang in der medizinischen Versorgung lediglich ein begrenzter Kreis an Patienten bzw. an Frakturarten durch die vorteilhafte Anwendung einer kompressionserzeugenden Fixierung begünstigt.

Der Erfindung liegt daher die Aufgabe zugrunde, ein Spannelement für eine Fixierungsvorrichtung bereitzustellen, das besonders kostengünstig herstellbar ist, um den Anwendungsbereich kompressionserzeugender Fixierungen zu erweitern, sowie ein entsprechendes Verfahren zu schaffen, mittels dem das Spannelement kostengünstig herstellbar ist. Des Weiteren liegt dabei die Aufgabe zugrunde eine Fixierungsvorrichtung bereitzustellen, bei der das Spannelement angewendet wird.

Die Aufgabe wird durch die Fixierungsvorrichtung gemäß Anspruch 1 sowie durch die Verfahren gemäß den Ansprüchen 15 oder 16 gelöst.

Das Spannelement zeichnet sich aus durch einen im Querschnitt im Wesentlichen hohlen, von einer umlaufenden Wandung, insbesondere mit im Wesentlichen gleichbleibender Wandstärke, umfassten, geschlossenen Konturkörper, mit zwei gegenüberliegenden stirnseitigen Aufnahmeabschnitten zur Aufnahme von Befestigungsmitteln, vorzugsweise Corticalisschrauben, die durch den Konturkörper hindurchsteckbar sind, und zwei Seitenflanken mit Beugeabschnitten, wobei die umlaufende Wandung des Konturkörpers wenigstens abschnittsweise, insbesondere im Bereich der stirnseitigen Aufnahmeabschnitte und im Bereich der seitlichen Beugeabschnitte, federelastisch verformbar ist, und wobei über die Beugeabschnitte ein definiertes elastisches Verhalten des Spannelements derart einprägbar ist, dass eine vorbestimmte Spannung zwischen den Aufnahmeabschnitten erzeugbar ist.

Die gewünschte Elastizität des Spannelements in dessen Längsrichtung steht direkt im Verhältnis mit einer Querkontraktion des Spannelements. Das Verhältnis zwischen Längsdehnung und Querkontraktion kann bei einem Spannelement mit einer z.B. rautenförmigen Geometrie auf einfache Weise über den Krümmungswinkel der Beugeabschnitte und der stirnseitigen Aufnahmeabschnitte beeinflusst werden. Aufgrund des einfachen Aufbaus des Spannelements kann die Fixierungsvorrichtung besonders kostengünstig hergestellt werden. Dadurch erweitert sich der potenzielle Anwendungsbereich kompressionserzeugender Fixierungen zur Beschleunigung des Heilungsprozesses einerseits hinsichtlich des potenziellen Patientenkreises und andererseits auch auf verhältnismäßig unkritische Knochenfrakturen.

Die Fixierungsvorrichtung zeichnet sich aus durch das Aufweisen von wenigstens einem Spannelement und wenigstens zwei Befestigungsmitteln, vorzugsweise Corticalisschrauben, die im Bereich der Bruchenden in den Knochen einbringbar sind.

Bei der Anwendung der Fixierungsvorrichtung ist vorgesehen, eine Kompression an einer Bruchstelle mittels eines erfindungsgemäßen Spannelements herzustellen. Dazu wird vor dem Einsetzen des Spannelements ein Druck auf die Seitenflanken bzw. die Beugeabschnitte aufgebracht, um eine Querkontraktion des Spannelements einzuleiten. Mit der bewirkten Querkontraktion geht eine Längsdehnung des Spannelements einher. Umschließt nun das Spannelement die Befestigungsmittel, bewirkt die Vorspannung der Längsdehnung eine Zugkraft zwischen den Befestigungsmitteln. Demzufolge bedarf es zur Erzeugung einer Kompression keiner zusätzlichen konstruktiven Mittel wie z.B. einer Zugfeder, oder einer auf Zugspannung justierten Schraubverbindung.

Gemäß einer Ausführungsform kann der geschlossene Konturkörper des Spannelements im Wesentlichen oval, rautenförmig, doppel-rautenförmig oder brillenförmig ausgebildet sein. Bei der Rautenform oder der ovalen Form weisen die Beugeabschnitte der Seitenflanken nach außen. Diese Bauform ermöglicht einen großen möglichen Bereich an Krümmungswinkeln.

Bei einer anderen Ausführungsform weisen die Beugeabschnitte nach innen, sodass sich die Breite des Spannelements zum mittleren Bereich des Spannelements hin verringert. Somit ergibt sich eine brillenförmige Gestalt des Spannelements, dessen Breite von dem Krümmungsradius der Aufnahmeabschnitte bzw. von der Breite eines aufzunehmenden Befestigungsmittels oder einer Spannhülse abhängt. Die schmale Bauform verringert den Platzbedarf im Körpergewebe und möglicht an Verknüpfungspunkten engere Ausrichtungswinkel zwischen benachbarten Spannelementen.

Bei einer weiteren Ausführungsform können die Seitenflanken jeweils drei gegenläufige Beugeabschnitte umfassen, wobei jeweils der mittlere Beugeabschnitt nach innen weist. Bei einer rautenförmigen oder brillenförmigen Ausführungsform ist die Gestalt des Spannelements, insbesondere bei Einhaltung eines funktionsoptimalen Krümmungswinkelbreichs der Beugeabschnitte, im Wesentlichen festgelegt. Die Anordnung mehrerer alternierender Beugeabschnitte ermöglicht eine größere Gestaltungsfreiheit bezüglich der Längen- und Breitenabmessung des Spannelements.

Bei einer weiteren Ausführungsform kann der geschlossene Konturkörper des Spannelements eine Krümmung in Bezug auf die Erstreckungsebene der Kontur aufweisen. Dabei kann die Krümmung insbesondere der Oberfläche des betreffenden Knochens entsprechen. Diese anatomische Anpassung ist z.B. bei Anwendungsbereichen wie der Kniescheibe oder einer radialen Befestigung auf einem Röhrenknochen zur Versorgung einer Torsionsfraktur mit einer schräg oder leicht spiralförmig verlaufenden Frakturlinie vorgesehen.

Zur Versorgung von Trümmerfrakturen oder von Knochen mit besonderer Anatomie ist herkömmlicherweise der Einsatz mehrerer einzelner oder individuell gestalteter Knochenplatten erforderlich. Eine Fixierung mit mehreren einzelnen Knochenplatten ist mit einer erhöhten Anzahl von Schrauben verbunden, die auf der begrenzten Fläche von Knochenfragmenten im Bereich der Bruchufer eingebracht werden müssen. Dadurch erhöht sich die Komplexität, der zeitliche Aufwand und das Risiko des operativen Eingriffs. Eine individuelle Anfertigung von Knochenplatten ist kostenintensiv und insbesondere mit Wartezeiten verbunden.

Beim vorliegenden erfindungsgemäßen Spannelement kann die Wandung im Bereich der Aufnahmeabschnitte Durchbrüche und/oder Ausschnitte aufweisen, die derart ausgebildet sind, dass durch eine Nut-Gestalt und/oder eine Feder-Gestalt von Wandungsabschnitten eine komplementäre Passung bereitgestellt ist, vermittels der sich die Konturen der benachbarte Spannelemente in Überschneidung bringen lassen.

Die Durchbrüche und Ausschnitte ermöglichen die Verknüpfung mehrerer Spannelemente im Sinne einer Nut und Feder-Verbindung. Durch die Kombination mehrerer Spannelemente, beispielsweise auch solcher mit verschiedenen Abmessungen, Krümmungen und Bauformen, ist somit eine modulare Konfiguration nach Art eines Baukastensystems vorgesehen, anhand der eine erfindungsgemäße Fixierungsvorrichtung auf einfache Weise an die Struktur eines Bruchs, insbesondere einer Trümmerfraktur, sowie an die lokalen anatomischen Begebenheiten individuell angepasst werden kann.

Gegenüber einer Vielzahl einzelner Knochenplatten lässt sich durch die Verknüpfung mehrerer Spannelemente die Anzahl der erforderlichen Befestigungspunkte verringern. Dies wirkt sich besonders bei kleinen Knochenfragmenten vorteilhaft aus und reduziert zudem den operativen Aufwand erheblich. Gegenüber individuell angefertigten Knochenplatten kann durch die modulare Konfigurationsmöglichkeit eine schnelle Versorgung von komplizierten Frakturen erreicht werden.

Bei einer weiteren Ausführungsform können an der Außenfläche der Wandung Kupplungselemente oder ein Kugelelement oder ein sphärisches Klemmelement vorgesehen sein, vermittels denen sich benachbarte Spannelemente, vorzugsweise kugelgelenkig gegeneinander drehbar und/oder zueinander lagefixiert, verbinden lassen. Diese Ausführungsformen stellen Möglichkeiten zur Verknüpfung mehrerer Spannelemente dar, die keine Überschneidung derselben erfordern und die in einer bestimmten Lage der Spannelemente zueinander fixiert werden können. Somit kann z.B. eine Verkettung von Spannelementen über die Seitenflanken realisiert werden. Die Ausführungsform eines verklemmbaren Kugelgelenks bietet hierbei eine große Flexibilität für eine multidirektionale Verknüpfung.

Bei einer weiteren Ausführungsform können an der Wandung Befestigungsaugen vorgesehen sein, durch welche die Befestigungsmittel hindurchsteckbar sind. Diese Ausgestaltung bietet eine weitere Möglichkeit zu einer einfachen Befestigung eines Spannelements.

Bei einer weiteren Ausführungsform können an zwei gegenüberliegenden Wandungsabschnitten aufeinander zuweisende Begrenzungsstege ausgebildet sein, deren Enden sich bei einer Verformung des Spannelements berühren. Die Begrenzung eines aufgrund äußerer Kräfte eingeleiteten Verformungswegs bietet einen Schutz gegen Verformungen, die über den elastischen Bereich des Spannelements hinausgehen und zu bleibenden Veränderungen der Ausgangsmaße des Spannelements führen würden. Zudem kann der Begrenzungsanschlag als Indikator einer definierten Vorspannung des Spannelements genutzt werden.

Bei einer anderen Ausführungsform können innerhalb des Konturkörpers an den gegenüberliegenden Beugeabschnitten zwei aufeinander zu gerichtete, miteinander in Eingriff bringbare Rastelemente, vorzugsweise ein hinterschnittenes Element und ein Fallenelement, angeordnet sein, vermittels denen die Beugeabschnitte in einer vorgespannten Lage des Spannelements rastend, wieder lösbar, verbindbar sind. Somit kann das Spannelement besonders anwendungsfreundlich vor einem operativen Eingriff in einer vordefinierten Vorspannung verrastet werden. Nach der Befestigung kann die Rastverbindung gelöst werden, sodass eine der Vorspannung entsprechende Zugkraft zwischen den Befestigungsmitteln anliegt.

Die erfindungsgemäße Fixierungsvorrichtung kann weiterhin wenigstens eine Spannhülse aufweisen, in der ein Befestigungsmittel aufnehmbar ist, wobei die Spannhülse vorzugsweise zwei stirnseitige Kröpfungen umfasst, die im Wesentlichen entsprechend einer Wandungshöhe des Spannelements beabstandet sind. Die Kröpfungen an den Stirnseiten der Spannhülse verhindern eine Verschiebung des Spannelements in axialer Richtung. Ferner weist die Spannhülse eine größere Umfangsfläche als z.B. ein Gewindeabschnitt eines Befestigungsmittels auf, wodurch die Kontaktfläche zwischen dem Befestigungsmittel und dem Spannelement vergrößert und eine entsprechende Punktbelastung reduziert wird. Infolgedessen kann ein potenzieller Materialabrieb und eine damit verbundene Kontaminierung des umgebenden Körpergewebes minimiert werden.

Die erfindungsgemäße Spannhülse kann zweiteilig ausgebildet und in axialer Richtung zusammenfügbar sein, sodass die Wandung des Spannelements zwischen den Kröpfungen einspannbar ist. Dabei wird ein fester Sitz zwischen Befestigungsmittel und Spannelement geschaffen und zudem verhindert, dass Körpergewebe zwischen diese beiden Elemente gelangt, eingeklemmt wird, oder einwächst.

Der Heilungsprozess einer Knochenfraktur kann weiterhin begünstigt werden, wenn die stützende Corticalisschicht des Knochens nach einer gewissen Zeit Belastungen ausgesetzt wird, um wieder eine tragende Funktion zu übernehmen, so wie dies beispielsweise auch nach der Abnahme einer Gipsschiene der Fall ist. Bei einer herkömmlichen Fixierung wird hingegen ein Teil der tragenden Funktion dauerhaft durch die starre Knochenplatte übernommen.

Gemäß eines Aspekts der Erfindung kann die Fixierungsvorrichtung Elemente aus einem resorbierbaren Kunststoff, d.h. einem Material, das von dem umgebenden Körpergewebe abgebaut wird, umfassen. Resorbierbare Kunststoffe weisen jedoch relativ spröde Materialeigenschaften auf. Sie sind daher nur bedingt zur Ausbildung von Bereichen geeignet, die mechanischen Belastungen ausgesetzt sind. Zudem sind derartige Materialien auch nicht zur Ausbildung elastischer Körper vorgesehen.

Die Spannhülse kann demnach in einer weiteren Ausführungsform radial in zwei Abschnitte aufgeteilt sein, wobei der radial innere Abschnitt oder der radial äußere Abschnitts aus einem resorbierbaren Kunststoff ausgebildet ist. Durch die Resorption einer der beiden Abschnitte entsteht ein Spiel zwischen dem Befestigungsmittel und dem Spannelement, das zu einer Kraftentkopplung zwischen den Befestigungsmitteln führt. Wenn vorzugsweise ein äußerer Abschnitt aus einem Metall und ein innerer Abschnitt aus einem resorbierbaren Kunststoff besteht, verbleibt mittels der Kröpfungen des äußeren Abschnitts noch eine lokale Eingrenzung des Spannelements. Hierdurch kann ein Lösen des Spannelements aus seiner Anordnung bzw. eine potenzielle Beeinträchtigen des angrenzenden Köpergewebes verhindert werden.

Alternativ kann die Fixierungsvorrichtung in einer anderen Ausführungsform mindestens ein Hülsenelement aufweisen, das aus einem resorbierbaren Kunststoff ausgebildet ist, wobei das Befestigungsmittel in dem Hülsenelement aufnehmbar ist. Durch die Resorption des Hülsenelements erfolgt dabei in der Fixierungsvorrichtung ebenso eine Kraftentkopplung zwischen den Befestigungsmitteln, wie vorstehend erwähnt. Die Kraftübertragung zwischen der Fixierungsvorrichtung und dem Knochen geht daher mit fortschreitender Resorption der Hülsenelemente verloren, wodurch die Belastungen auf den Knochen wieder ansteigen.

Bei einer wiederum anderen Ausführungsform der Fixierungsvorrichtung kann zwischen den Beugeabschnitten ein Inlay eingespreizt sein, dessen Länge die Beugeabschnitte gegen eine Vorspannung des Spannelements auseinander drückt, wobei das Inlay aus einem resorbierbaren Material besteht. Das Inlay hält das Spannelement unter Vorspannung. Wenn das Inlay während des Heilungsprozesses resorbiert wird, kehrt das Spannelement in seine Ausgangslage zurück, wobei sich der Abstand zwischen den Aufnahmeabschnitten wieder vergrößert und eine Kraftübertragung zwischen den Befestigungsmitteln, hinsichtlich einer Zugkraft, verloren geht.

Gemäß einer beispielgebenden Ausführungsform können sich bei der Fixierungsvorrichtung benachbarte Spannelemente derart überschneiden, dass das Befestigungsmittel, die Spannhülse oder das Hülsenelement von den stirnseitigen Aufnahmeabschnitten der überschneidenden Spannelemente umschließbar ist. Somit sind Fixierungsvorrichtungen aus einer kettenförmigen oder einer sternförmigen Anordnung von Spannelementen realisierbar. Durch eine Kettenanordnung kann bei einer geringen Anzahl von Befestigungsmitteln eine Kompression zwischen mehreren Knochenfragmenten erzielt, oder längere Distanzen bei einer geringen Breitenabmessung das Spannelement überbrückt werden. Durch eine Sternanordnung von Spannelementen kann eine mehrdirektionale Kompression auf Knochenfragmente mit überkreuzenden Frakturlinien, wie z.B. bei einer Trümmerfraktur und insbesondere einer Keilfraktur, aufgebracht werden. Durch die Wahl der Anordnung von Spannelementen und der Verknüpfungspunkte lässt sich die Fixierungsvorrichtung individuell auf den Verlauf verschiedener Bruchufer ausrichten.

Insbesondere kann bei einer Ausführungsform ein Innenradius der stirnseitigen Aufnahmeabschnitte des Spannelements dem Außenradius eines Umfangsflächenabschnitts eines Befestigungsmittels, eines Hülsenelements oder einer Spannhülse entsprechen. Durch eine möglichst große Kontaktfläche zwischen dem inneren Krümmungsradius des Spannelements und dem Außenumfang des Befestigungsmittels kann die Punktbelastung zwischen den Elementen verringert werden. Das führt, wie vorstehend beschrieben, zu einer Minimierung einer potenziellen Kontaminierung des umgebenden Körpergewebes durch Materialabrieb. Des Weiteren erfordert ein sicherer Sitz des Befestigungsmittels an dem Spannköper aufgrund der Zugspannung keine umschließende Aufnahme. Somit entfällt zugunsten einer einfachen Bauform des Spannelements das Erfordernis von Aufnahmebohrungen oder sonstigen umschließenden Aufnahmen.

Erfindungsgemäß kann das Spannelement aus einem ausgewählten Hohlprofil bzw. einem im Wesentlichen hohlen Profil hergestellt werden, das stranggepresst wird. Das Spannelement wird durch einen Hohlprofilabschnitt gebildet, der an einem in Längsrichtung des Hohlprofils vorbestimmten Querschnitt, der die Höhe des Spannelements bildet, abgetrennt wird. Außerdem können Ausschnitte oder Durchbrüche in der Wandung des Hohlprofilabschnitts abgetragen werden. So kann das Spannelement mit einem besonders geringen Aufwand hergestellt werden. Das Verhältnis von Höhe zu Wandungsdicke bietet eine Möglichkeit, die Elastizität und die Steifigkeit bezüglich der Erstreckungsebene der Kontur des Spannelements auszuwählen.

Wahlweise kann das Spannelement aus einem ausgewählten Vollprofil hergestellt werden, das mittels Strangpressverfahren gegossen wird. Das Spannelement wird durch einen Vollprofilabschnitt gebildet, der an einem in Längsrichtung des Vollprofils vorbestimmten Querschnitt, der die Höhe des Spannelements bildet, abgetrennt wird. Danach wird das Vollmaterial zur Ausbildung eines im Querschnitt im Wesentlichen hohlen, von einer umlaufenden Wandung umfassten, geschlossenen Konturkörpers spanabhebend bearbeitet. Außerdem können Ausschnitte oder Durchbrüche in der Wandung des geschlossenen Konturkörpers abgetragen werden. Auf diese Weise können bei verhältnismäßig geringem Aufwand besondere Ausführungsformen des Spannelements hergestellt werden.

Weitere Ausführungsformen, Merkmale und Vorteile der Erfindung werden anhand der nachfolgenden Beschreibung von Ausführungsformen unter Bezugnahme auf die Figuren ersichtlich. Dabei zeigt:

Fig. 1         eine perspektivische Ansicht eines erfindungsgemäßen Spannelements;

Fig. 2         eine perspektivische Ansicht eines Spannelements und einer Corticalisschraube mit einer Spannhülse als Befestigungsmittel;

Fig. 3         eine perspektivische Ansicht eines Spannelements und einer zweiteiligen Spannhülse;

Fig. 4         eine perspektivische Ansicht eines brillenförmigen Spannelements;

Fig. 5         eine perspektivische Ansicht eines doppelrautenförmigen Spannelements;

Fig. 6         eine perspektivische Ansicht eines Spannelements, das eine Krümmung in der Erstreckungsebene der Kontur aufweist;

Fig. 7         eine Detailansicht einer Verknüpfung mit überschneidenden Spannelementen;

Fig. 8         eine perspektivische Ansicht einer als Kettenanordnung ausgeführten Fixierungsvorrichtung;

Fig. 9         eine perspektivische Ansicht einer als Sternanordnung ausgeführten Fixierungsvorrichtung;

Figuren 10 A-C    perspektivische Ansichten von Spannelementen mit Kupplungselementen;

Figuren 11 A-C    perspektivische Ansichten eines Spannelements mit einer kugelgelenkigen Klemmverbindung;

Figuren 12 A-B    perspektivische Ansichten von Spannelementen mit Befestigungsaugen;

Fig. 13        eine perspektivische Ansicht eines Spannelements mit Begrenzungsstegen;

Fig. 14        eine perspektivische Ansicht einer Fixierungsvorrichtung mit resorbierbaren Hülsenelementen;

Fig. 15        eine perspektivische Ansicht einer radial aufgeteilten Spannhülse mit einem resorbierbaren Abschnitt;

Fig. 16        eine perspektivische Ansicht eines Spannelements mit einem Inlay;

Fig. 17        eine Draufsicht auf ein Spannelement mit Rastelementen.

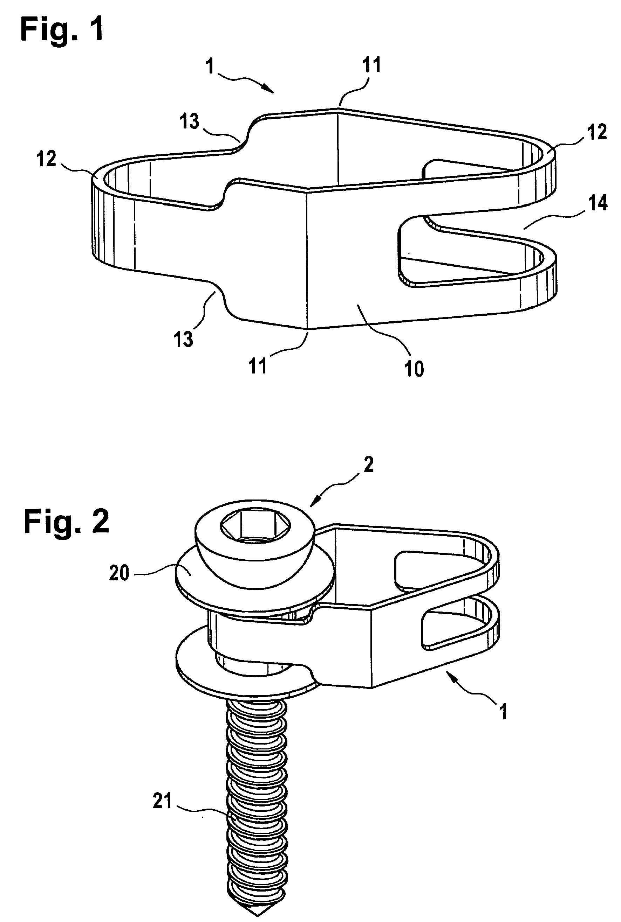

Fig.1 zeigt ein Spannelement 1 mit einer umlaufenden Wandung 10, die zwei Beugeabschnitte 11 und zwei Aufnahmeabschnitte 12 umfasst. Die zwei Aufnahmeabschnitte 12 liegen einander an den Stirnseiten des Spannelements 1, d.h. in einer Längsrichtung zwischen zwei Befestigungsmitteln (nicht dargestellt), gegenüber und weisen eine nach außen weisende Krümmung auf. Die zwei Beugeabschnitte 11 liegen einander an den Seitenflanken des Spannelements 1 in einer Breitenrichtung, d.h. senkrecht zu der Längsrichtung, gegenüber und weisen eine nach außen weisende Krümmung auf. Das in Fig.1 gezeigte Spannelement 1 ist im Wesentlichen rautenförmig ausgebildet.

Wenn sich der Krümmungsradius der Beugeabschnitte 11 über die gesamte Länge derselben erstreckt, kann das Spannelement 1 ebenso eine im Wesentlichen ovale Form aufweisen.

Der Krümmungsradius der Aufnahmeabschnitte 12 ist dabei beispielsweise derart gewählt, dass er mit einem Befestigungsmittel bzw. einer Spannhülse, oder ggf. mit einem Hülsenelement korrespondiert und dieses flächig aufnimmt. Aufgrund der anliegenden Zugspannung ist somit eine formschlüssige Aufnahme des Befestigungsmittels bzw. der Spannhülse gewährleistet.

Das Spannelement 1 kann eine einheitliche umlaufende Wandungsdicke aufweisen. Diese Ausgestaltung begünstigt u.a. eine Herstellung des Spannelements 1 durch Biegeformen einer mittels Schweißnaht zu verbindenden Metallbandschleife oder durch Umformen eines Profils mit Schweißnaht.

Die oberen und unteren Kanten der Wandung sind abgerundet oder angefast, um eine Irritation des umgebenden Körpergewebes gering zu halten.

Die Wandung 10 des Spannelements 1 kann weiterhin eine einheitliche Höhe aufweisen, sodass das Spannelement 1 in Form eines Profilabschnitts mittels eines Querschnitts aus einem Profil, z.B. einem Strangpressprofil, abgetrennt werden kann. Dies kann auf besonders kostengünstige Weise bei einem Hohlprofil mit einer geeigneten Kontur und Wandungsdicke vorgenommen werden, da hierbei lediglich die Bestimmung der Wandungshöhe durch Ansetzen des abtrennenden Querschnitts verbleibt. Ferner kann das Spannelement 1 durch spanabhebende Bearbeitung aus einem Vollprofilabschnitt gefertigt werden, um weitere Ausgestaltungen, wie z.B. Kupplungselemente oder Begrenzungsstege, integral und individuell auszubilden.

Das Spannelement 1 wird aus biokompatiblen Materialen hergestellt. Zudem ist ebenfalls ein Hybridaufbau möglich, bei dem die Wandung der Beugeabschnitte 11 aus einem Material mit der gewünschten elastischen Eigenschaft bestehen und weitere Bereiche des Spannelements 1, wie z.B. die Aufnahmeabschnitte 12, aus einem steifen oder resorbierbaren Material gebildet werden. Ein Hybridaufbau lässt sich ebenso nach einem oben genannten Verfahren herstellen, insbesondere durch Auswahl eines entsprechenden Kompositprofils, das über den Querschnitt betrachtet aus Abschnitten verschiedener Werkstoffe zusammengesetzt ist.

Als Ausgangsmaterial für das Spannelement 1 kommen beispielsweise Metalle aus der Gruppe: X42CrMo15, X100CrMo17, X2CrNiMnMoNNb21-16-5-3, X20Cr13, X15Cr13, X30Cr13, X46Cr13, X17CrNi16-2, X14CrMoS17, X30CrMoN15-1, X65CrMo 17-3, X55CrMo14, X90CrMoV18, X50CrMoV15, X 38CrMo V15, G-X 20CrMo13, X39CrMo17-1, X40CrMoVN16-2, X105CrMo17, X20CrNiMoS13-1, X5CrNi18- 0, X8CrNiS18-9, X2CrNi19-11, X2CrNi18-9, X10CrNi18-8, X5CrNiMo17-12-2, X2CrNiMo17-12-2, X2CrNiMoN25-7-4, X2CrNiMoN17-13-3, X2CrNiMo17-12-3, X2CrNiMo18-14-3, X2CrNiMo18-15-3; X 2 CrNiMo 18 14 3, X13CrMnMoN18-14-3, X2CrNiMoN22136, X2CrNiMnMoNbN21-9-4-3, X4CrNiMnMo21-9-4, X105CrCoMo18-2, X6CrNiTi18-10, X5CrNiCuNb16-4, X3CrNiCuTiNb12-9, X3CrNiCuTiNb12-9, X7CrNiA117-7, CoCr20Ni15Mo, G-CoCr29Mo, CoCr20W15Ni, Co-20Cr-15W-10Ni, CoCr28MoNi, CoNi35Cr20Mo10, Ti1, Ti2, Ti3, Ti4, Ti-5Al-2,5Fe, Ti-5Al-2,5Sn, Ti-6Al-4V, Ti-6Al-4V ELI, Ti-3Al-2,5V (Gr9), 99,5Ti, Ti-12Mo-6Zr-2Fe, Ti-13,4Al-29Nb, Ti-13Nb-13Zr, Ti-15Al, Ti-15Mo, Ti- 15Mo-5Zr-3Al, Ti-15Sn, Ti-15Zr-4Nb, Ti15Zr-4Nb-4Ta, Ti-15Zr-4Nb-4Ta-0,2Pd, Ti-29Nb-13Ta-4,6Zr, Ti-30Nb-10Ta-5Zr, Ti-35,5Nb-1,5Ta-7,1Zr, Ti-35Zr-10Nb, Ti-45Nb, Ti-30Nb, Ti-30Ta, Ti-6Mn, Ti-5Zr-3Sn-5Mo-15Nb, Ti-3Al-8V-6Cr-4Zr-4Mo, Ti-6Al-2Nb-1Ta-0,8Mo, Ti-6Al-4Fe, Ti-6Al-4Nb, Ti-6Al-6Nb-1Ta, Ti-6Al-7Nb, Ti-6Al-4Zr-2Sn-2Mo, Ti-8,4Al-15,4Nb, Ti-8Al-7Nb, Ti-8Al-1Mo-1V, Ti-11Mo-6Zr-4Sn, in Betracht.

Ferner kommen Polymere aus der Gruppe: MBS, PMMI, MABS, CA, CTA, CAB, CAP, COC, PCT, PCTA, PCTG, EVA, EVAL, PTFE, ePTFE, PCTFE, PVDF, PVF, ETFE, ECTFE, FEP, PFA, LCP, PMMA, PMP, PHEMA, Polyamid 66, Polyamid 6, Polyamid 11 , Polyamid 2, PAEK, PEEK, PB, PC, PPC, PETP, PBT, MDPE, LDPE, HDPE, UHMWPE, LLDPE, PI, PAI, PEI, PIB, POM, PPO, PPE, PPS, PP, PS, PSU, PESU, PVC, PVC-P, PVC-U, ABS, SAN, TPE-U, TPE-A, TPE-E, PVDC, PVA, SI, PDMS, EPM, EP, UF, MF, PF, PUR, UP, PEBA, PHB, PLA, PLLA, PDLA, PDLLA, PGL, PGLA, PGLLA, PGDLLA, PGL-co-poly TMC, PGL-co-PCL, PDS, PVAL, PCL, Poly-TMC, PUR (linear), NiTi Superelastic, NiTi Shape Memory, in Betracht.

Des Weiteren kommen Keramiken aus der Gruppe: $Al_2O_3$ (Aluminiumoxid), Y-TZP (Zirkonoxidkeramik), AMC (Alumina Matrix Composite), HA (Hydroxilapatit), TCP (Tricalciumphosphat), Ceravital (Glaskeramik/Bioglas®), FZM/K (Zirkonoxid, teilstabilisiert), TZP-A (Zirkonoxidkeramik), ATZ (Alumina-toughened Zirconia), C799 (Aluminiumoxidkeramik), Schott 8625 (Transponderglas), in Betracht.

Darüber hinaus kommen Kombinationen hiervon in Betracht.

Die in Fig. 1 dargestellte Rautenform für das Spannelement 1 weist günstige Eigenschaften hinsichtlich der Längsdehnung und Querkontraktion des Spannelements 1 auf, da sich ein elastisches Verhalten in Längsrichtung des Spannelements 1 durch das Seitenverhältnis der parallel zueinander verlaufenden Wandabschnitte vorbestimmen lässt.

Bei der Streckung eines rautenförmigen Spannelements 1 bzw. im Falle eines entgegengerichteten Drucks auf die Beugeabschnitte 11 gilt annäherungsweise ein Verhältnis zwischen Längsdehnung und Querkontraktion von:

$$\Delta l = \sqrt{4x^2 - (b - \Delta b)^2} - \sqrt{4x^2 - b^2} \ ,$$

wobei b die Breite des Spannelements 1 in der Ausgangslage ist; $\Delta b$ die negative Längenveränderung in Breitenrichtung ist; $\Delta l$ die positive Längenveränderung in Längsrichtung ist; und x der näherungsweise konstant bleibende Abstand zwischen dem Krümmungscheitelpunkt eines Aufnahmeabschnitts 12 und dem Krümmungscheitelpunkt eines Beugeabschnitts 11 ist.

Zudem kann das elastische Verhalten des Spannelements 1 durch geeignete Auswahl des Wandungsquerschnitts und des Materials beeinflusst werden. Hierbei hängt das elastische Verhalten von dem materialspezifischen Elastizitätsmodul und der spezifischen inneren Materialreibung des gewählten Materials ab.

Unter Berücksichtigung der inneren Materialreibung, die nach Entnahme einer Vorspannkraft verbleibt, herrscht somit annähernd eine Zugkraft von:

$$Fl = \frac{Fb}{k} - Freib$$

wobei *Fl* die Zugkraft in Längsrichtung ist; *Fb* die aufgebrachte Vorspannkraft in Breitenrichtung ist; *Freib* die spezifische innere Materialreibung ist; und *k eine* Federkonstante hinsichtlich des materialspezifischen Elastizitätsmoduls und der Geometrie ist.

Wie in Fig. 2 erkennbar ist, umfasst die Fixierungsvorrichtung zu dem Spannelement 1 auch ein Befestigungsmittel 2. Das Befestigungsmittel 2 kann ein chirurgisches Implantat wie z.B. ein Knochennagel, eine Knochenschraube und insbesondere eine Corticalisschraube 21 sein, das in den Knochen eingebracht wird. Die Corticalisdicke der verschiedenen Knochen weicht hierbei je nach Knochen, z.B. an einem Oberschenkelknochen oder einem Kieferknochen, erheblich ab. Für die verschiedenen Anwendungen sind nach ISO 5835: 1991 oder ASTM: F 543-07 genormte Corticalisschrauben 21 mit gestaffelten Durchmessern und Längen erhältlich.

Grundsätzlich sind hierbei jedoch keine festgelegten Vorgaben zwischen der Größe des Spannelements 1 und dem betreffenden Knochen vorgesehen. Allerdings ist eine proportionale Dimensionierung des Spannelements 1 zur dem verwendeten Schraubensystem denkbar. Aus der praktischen Medizintechnik kann nährungsweise ein Zusammenhang zwischen dem Nenndurchmesser der Corticalis- bzw. Spongiosaschraube und dem Anwendungsbereich hergestellt werden, der in folgender Tabelle aufgeführt ist.

| Schraubensystem | Einsatzbereiche |
|---|---|
| HA 1,5 Corticalis | MGK-Chirurgie |

(fortgesetzt)

| Schraubensystem | Einsatzbereiche |
|---|---|
| HA 2,0 Corticalis | Cranio; Fußchirurgie; Handchirurgie |
| HA 2,5 Corticalis | Cranio; Fußchirurgie; Trauma |
| HA 3,5 Corticalis | Thorax; Unterer Rücken; Armchirurgie |
| HA 4,0 Corticalis | Thorax; Wirbelsäule; Hüft- und Beckenbereich |
| HA 4,5 Corticalis | Beinchirurgie; Rumpf; Fibula; Schulter |
| HA 5,0 Corticalis | Beinchirurgie; Tibia; Femur |
| HB 4,0 Spongiosa | abhängig vom Belastungsfall |
| HB 6,0 Spongiosa | abhängig vom Belastungsfall |

Die Wahl der Dimensionierung und Positionierung des Schraubensystems sowie des Spannelements 1 hängt zudem von der Frakturart (z.B. Querfraktur, Schrägfraktur) und dem Frakturort ab, wodurch sich verschiedene Belastungsfälle ergeben.

So kommen bei Schaftfrakturen zumeist Corticalissysteme zum Einsatz, da hier kein Spongiosaanteil vorhanden ist. Bei gelenknahen Frakturen kommen hingegen oftmals Spongiosasysteme zum Einsatz, da der Anteil der Spongiosa in diesem Bereich sehr hoch ist. Spongiosaschrauben weisen einen höheren Traganteil auf, da diese bei demselben Nenndurchmesser einen größeren Kerndurchmesser aufweisen. Bei Gelenkfrakturen, d.h. Frakturen mit Beteiligung der Gelenkfläche, werden in Abhängigkeit der lokalen anatomischen Bedingungen sowohl Cortikalissysteme als auch Spongiosasysteme verwendet. Ebenso können bei einer mehrfachen Fraktur beide Systeme eingesetzt werden.

Zudem können neben einer einfachen, d.h. monocorticalen Fixationsmöglichkeit auch bicorticale Systeme vorgesehen sein, bei denen die Schraube durch den Knochen hindurch an beiden corticalen Bereichen fixiert wird.

Der Einsatz des Spannelements 1 zur Fixierung einer Bruchstelle kann insbesondere wie nachstehend beschrieben erfolgen.

Die Beugeabschnitte 11 des Spannelements 1 werden von Außen zusammen gedrückt, wodurch das Spannelement 1 eine Querkontraktion erfährt. Dabei tritt eine Längsdehnung des Spannelements 1 auf, d.h. der Abstand zwischen den Aufnahmeabschnitten 12 wird größer. Anschließend werden zwei Befestigungsmittel, beispielsweise Corticalisschrauben, hindurch gesteckt, so dass diese bei dem vergrößerten Abstand der Aufnahmeabschnitte 12 an dessen Innenflächen anliegen. In dieser Position werden die Befestigungsmittel in gegenüberliegende Bruchenden eines Knochens eingebracht. Nach Wegnahme der äußeren Kraft wird das Spannelement 1 aufgrund der Befestigungsmittel in der längsgedehnten Form, d.h. unter Vorspannung, gehalten. Diese Vorspannung übt dauerhaft eine Zugkraft zwischen den Befestigungsmitteln aus. Die Zugkraft zwischen den Befestigungsmitteln wird bei der Fixierungsvorrichtung zur Adaption der Bruchenden an einer Knochenfraktur angewendet.

Des Weiteren ist bei dem in Fig. 1 dargestellten Spannelement 1 durch einen oberen und unteren Ausschnitt 13 eine Art Feder in einen Wandungsabschnitt im Bereich eines Aufnahmeabschnitts 12 ausgebildet. Komplementär hierzu ist im Bereich des gegenüberliegenden Aufnahmeabschnitts 12 durch einen mittigen Durchbruch 14 eine Art Nut in einem Wandungsabschnitt ausgebildet. Die Feder-Gestalt und die Nut-Gestalt lassen sich ineinander fügen, sodass sich die Konturen der Wandungen 10 zweier benachbarter Spannelemente 1 überschneiden können. Die Ausschnitte 13 und die Durchbrüche 14 erstrecken sich soweit in Längsrichtung des Spannelements 1, dass in dem Überschneidungsbereich von zwei oder mehreren Spannelementen 1 dasselbe Befestigungsmittel 2 oder dieselbe Spannhülse von den Aufnahmeabschnitten 12 der Spannelement 1 vollständig umschlossen werden kann. Somit lassen sich verschiedene Spannelemente 1 nach dem Prinzip eines modularen Baukastensystems miteinander verknüpfen.

Ferner kann das Befestigungsmittel 2 einen Abschnitt mit einer Spannhülse 20 umfassen. Die Spannhülse 20 weist nach oben und unten eine stirnseitige Kröpfung auf, die eine Bewegung des Spannelements 1 in axialer Richtung des Befestigungsmittels 2 verhindert. Die Spannhülse 20 ist beispielsweise separat ausgebildet und wird mit einer Corticalisschraube 21 kombiniert, die in einer Durchgangsbohrung der Spannhülse 20 aufgenommen wird.

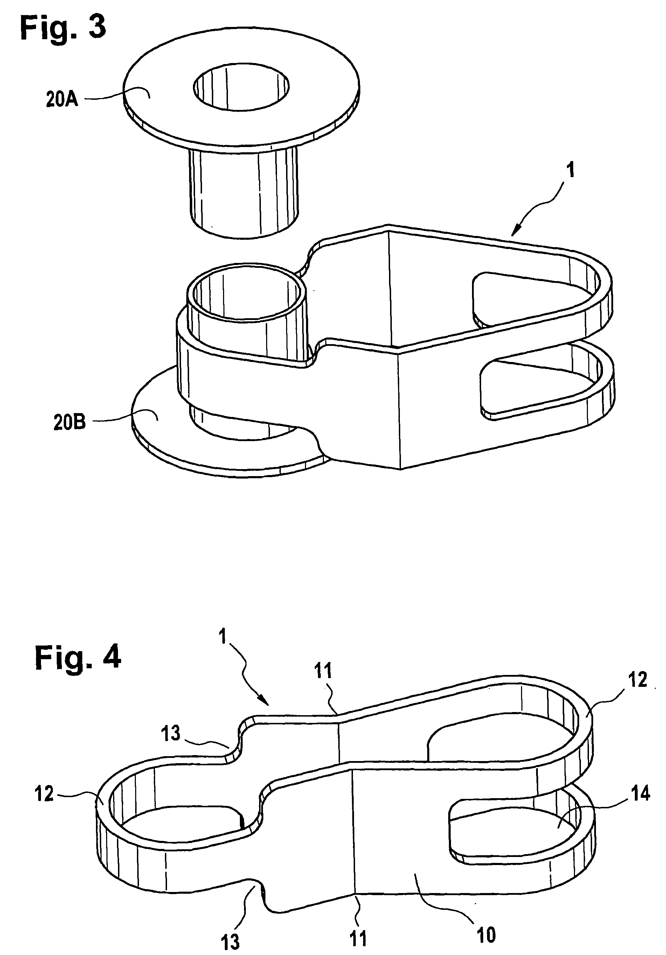

Bei der Ausführungsform in Fig. 3 ist die Spannhülse 20 zweiteilig ausgebildet, wobei sich die beiden Hälften 20A und 20B der Spannhülse 20 in axialer Richtung des Befestigungsmittels 2 zusammengefügt werden. Somit lässt sich ein Wandungsabschnitt des Spannelements 1 zwischen den stirnseitigen Kröpfungen der Hälften 20A und 20B der Spannhülse 20 einklemmen.

Das in Fig. 4 abgebildete Spannelement 1 umfasst zwei nach innen weisende Beugeabschnitte 11. In diesem Fall wird eine Längendehnung zur Vorspannung des Spannelements 1 durch ein Auseinanderdrücken der Beugeabschnitte 11 erzielt. Die sonstige Wirkungsweise entspricht derjenigen des obenstehend beschriebenen Spannelements 1 in Fig. 1.

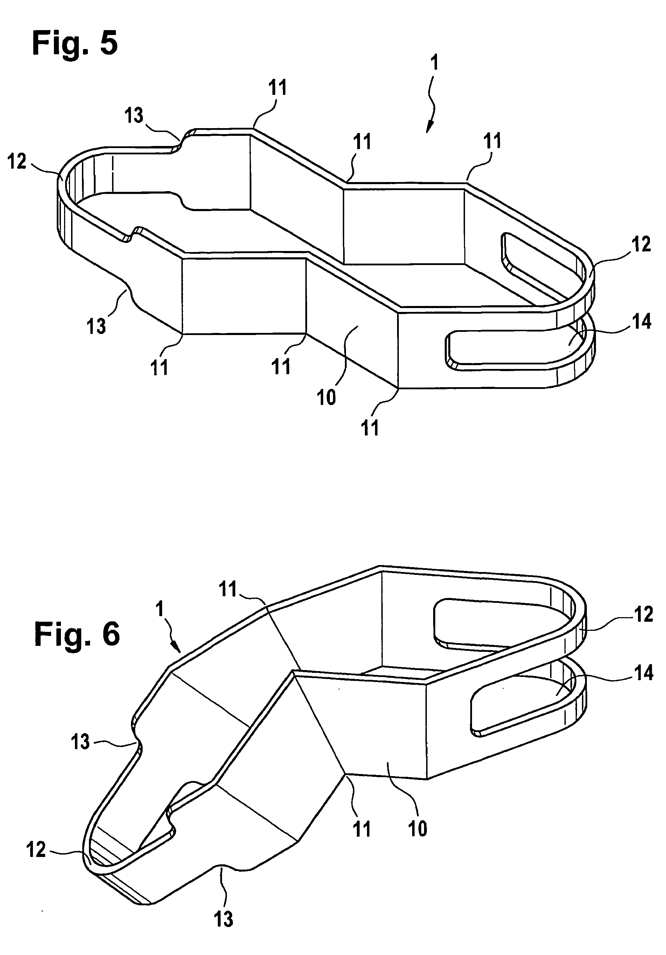

Der Fig. 5 ist ein Spannelement 1 zu entnehmen, welches an den Seitenflanken jeweils drei alternierende Beugeabschnitte 11 aufweist. Durch die Aneinanderreihung mehrerer gleicher Krümmungswinkel summiert sich eine Vorspannung über die einzelnen Beugeabschnitte 11 zu einer gesamten Längsdehnung des Spannelements 1 auf. Somit ist z.B. bei gleichem Elastizitätsmodul, Wandungsquerschnitt und Krümmungswinkel eine schmalere bzw. längere Bauform des Spannelements 1 realisierbar, ohne das elastische Verhalten in Längrichtung des Spannelements 1 wesentlich zu verändern. Die sonstige Handhabung und Wirkungsweise entspricht im Wesentlichen derjenigen der Ausführungsformen in den Figuren 1 und 4.

Das Spannelement 1 in Fig. 6 weist eine Krümmung in der Erstreckungsebene der Kontur auf und stellt somit eine von zahlreichen alternativen Bauformen zur anatomischen Anpassung dar. Das Spannelement 1 kann ebenso in Bezug auf eine oder mehrere beliebige Achsen oder Punkte gekrümmt sein. Die anatomischen Anpassungen können sowohl Verformungen die Erstreckungsebene der Kontur als auch die Wandungshöhe des Spannelements 1 betreffen.

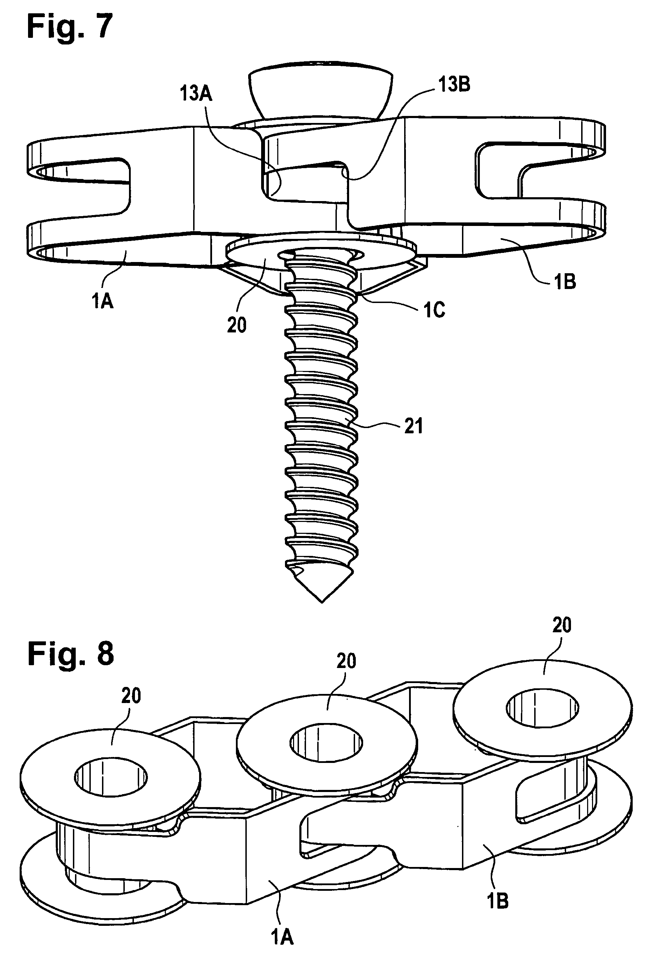

Wie Fig. 7 zu entnehmen ist, kann ein Ausschnitt 13 in der Wandung 10 auch bezüglich der Wandungshöhe versetzt angeordnet sein. Bei der abgebildeten Ausführungsform einer Verknüpfung überschneiden sich drei Federn der Spannelemente 1A-C und umschließen dieselbe Spannhülse 20. Dabei verlaufen die Feder-Gestalten bündig mit der Oberseite bzw. Unterseite der Spannelemente 1A und 1B. Die Spannelemente 1A und 1B können identisch sein, wobei eines der beiden umgekehrt angeordnet ist. Die Feder des Spannelements 1C verläuft mittig zu der Wandungshöhe und wird zwischen den Federn der Spannelemente 1A und 1B aufgenommen.

Die in Fig. 7 gezeigte Stapelanordnung besteht aus übereinanderliegenden Feder-Gestalten der Spannelemente 1A-C und umfasst somit keine Nut. Es sind jedoch ebenso Ausführungsformen von Verknüpfungspunkten mit einer beliebigen Anzahl und Kombination von Feder-Gestalten mit oder ohne Nut realisierbar. Vorteilhafterweise sind die Höhe und Position der betreffenden Feder-Gestalten und Nut-Gestalten in Abhängigkeit von der Anzahl der überschneidenden Spannelemente 1 derart ausgebildet, dass sich diese im Überschneidungsbereich gegenseitig zu einer Stapelhöhe ergänzen, die der Höhe eines Spannelements 1 entspricht.

Zur Überbrückung längerer Distanzen können mehrere Spannelemente 1 in einer Kette angeordnet werden. Wie in Fig. 8 dargestellt, sind für diese Ausführungsform vorzugsweise Spannelemente 1 mit einer Feder-Gestalt einerseits und einer komplementären Nut-Gestalt andererseits vorgesehen. Eine Kettenanordnung ist jedoch ebenso mit einer Überschneidung von zwei umgekehrten Feder-Gestalten realisierbar.

Zur Verknüpfung können die Spannelemente lediglich eine Spannhülse 20, d.h. ohne eine Corticalisschraube 21 oder dergleichen, umschließen. Somit kann die Zugkraft der vorgespannten Spannelemente 1 einer Kettenanordnung z.B. zwischen zwei Befestigungsmitteln an den beiden Enden der Kettenanordnung wirken. Bei dieser Ausführungsform ist eine zweiteilige Spannhülse 20 vorgesehen, dessen Hälften 20A und 10B beispielsweise mittels einer Presspassung verbunden werden.

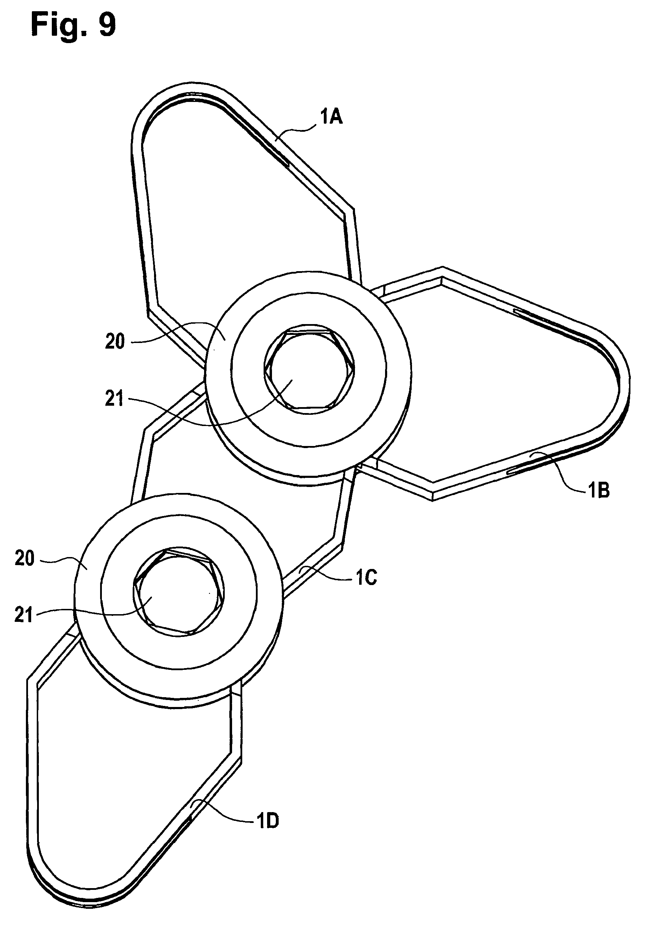

Mit dem modularen Aufbau der vorliegenden Fixierungsvorrichtung kann ebenso eine sternförmige Anordnung oder eine beliebige Kombination aus Ketten- und Sternanordnungen realisiert werden, wie dies in Fig. 9 dargestellt ist. Je nach Krümmungswinkel der Aufnahmeabschnitte 12 und der Breite der überschneidenden Spannelemente 1 kann eine Sternanordnung von drei oder mehr Spannelementen 1 gebildet werden. Auch bei dieser Verknüpfung kann eine Spannhülse 20 mit oder ohne einer eingeführten Corticalisschraube 21 oder dergleichen von den Spannelementen 1 umschlossen werden.

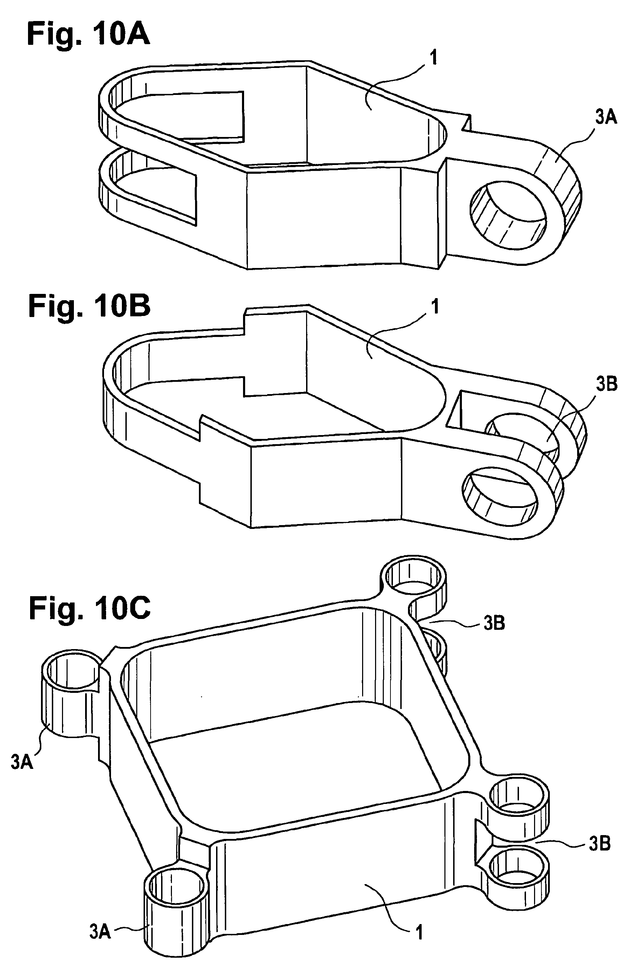

Eine Verknüpfung benachbarter Spannelemente 1 kann ebenfalls über Kupplungselemente 3A und 3B hergestellt werden, die an der Außenfläche der Wandung 10 ausgebildet sind. Hierzu können die Kupplungselemente die Form einer Öse bzw. einer doppelten Öse mit einem Aufnahmespalt aufweisen, die ebenfalls im Sinne einer Nut und Feder-Verbindung zusammengefügt werden können. Der Abstand des Aufnahmespalts in dem Kupplungselement 3B entspricht dabei vorteilhafterweise der Dicke des Kupplungselements 3A. Die ösenförmigen Kupplungselemente 3A und 3B weisen weiterhin ein Loch auf, durch welches sie im zusammengefügten Zustand mittels eines Bolzens, einer Schraube oder dergleichen durchgriffen werden können. Das Loch der Kupplungselemente 3A und 3B kann, wie in den Figuren 10 A und B dargestellt, in Breitenrichtung des Spannelements 1, oder wie in Fig. 10 C dargestellt, senkrecht zur Erstreckungsebene der Kontur, oder aber in einer beliebigen anderen Richtung, verlaufen.

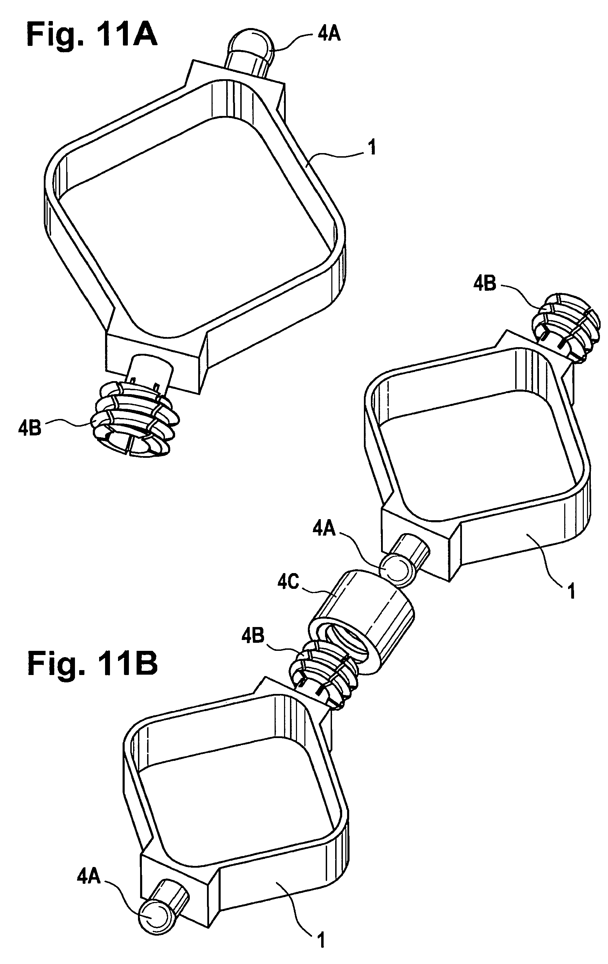

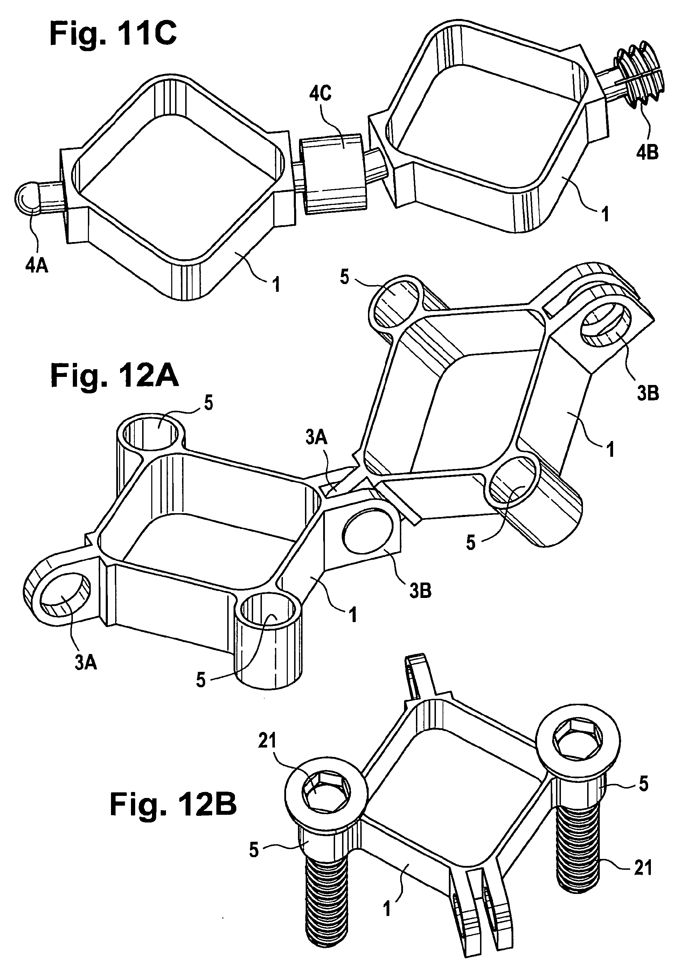

Wie den Figuren 11 A bis C zu entnehmen ist, können die Spannelemente 1 ebenso durch eine kugelgelenkige Klemmverbindung verknüpft werden. Hierfür ist an der Außenfläche der Wandung 10 des Spannelements 1 ein Kugelelement 4A und/oder ein sphärisches Klemmelement 4B ausgebildet. Das Klemmelement 4B weist eine sphärische Innenfläche mit Dehnungsfugen sowie ein Außengewinde auf, an dem es durch eine Mutter 4C verklemmt werden kann. Wenn das Kugelelement 4A mit Hilfe der Dehnungsfugen in das Klemmelement 4B eingespreizt ist, kann das Klemmelement 4B durch eine Gewindemutter auf der Außenfläche wahlweise soweit verspannt werden, dass ein Ausgleiten des Kugelelements 5B verhindert wird, oder dass die Verbindung lagefixiert ist.

Wie in Fig. 12 A und 12 B dargestellt ist, kann ein Spannelement 1 alternativ über Befestigungsaugen 5 an einem Knochen befestigt werden, in denen eine Corticalisschraube 21 oder dergleichen aufgenommen wird. In diesem Fall kann eine Spannhülse 20 entfallen. Die Befestigungsaugen sind an der Außenfläche der Wandung 10 des Spannelements 1 ausgebildet.

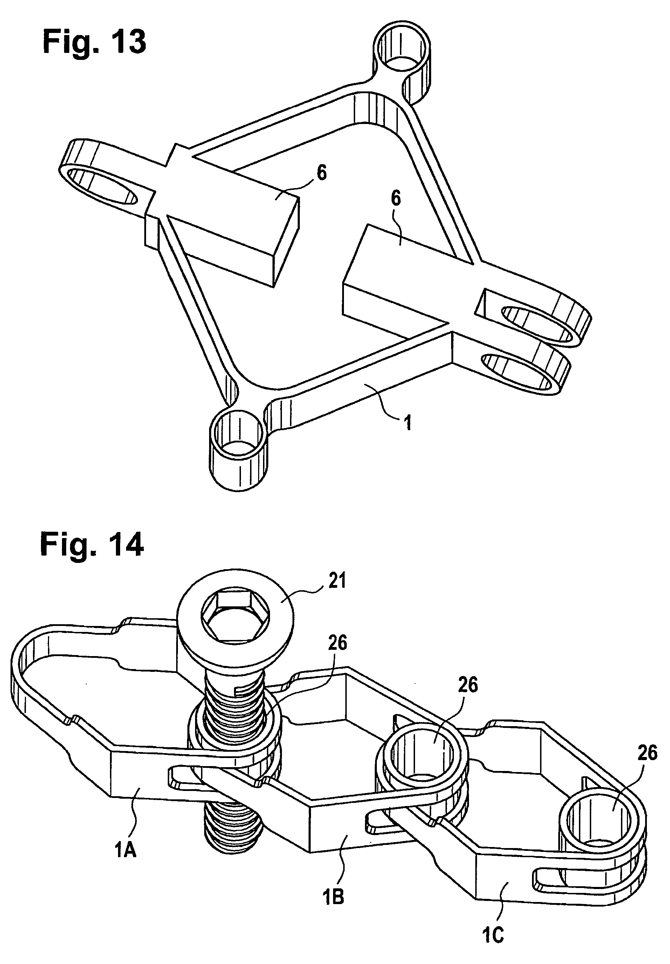

Wie in Fig. 13 dargestellt ist, können bei einem Spannelement 1 an zwei gegenüberliegenden Wandungsabschnitten Begrenzungsstege 6 ausgebildet sein, die sich von der Innenfläche der Wandung 10 des Spannelements 1 zu dessen Mittelpunkt erstrecken. Wenn sich das Spannelement 1 aufgrund einer von außen eingeleiteten Kraft elastisch verformt, bewegen sich die Enden der Begrenzungsstege 6 auf einander zu. Das elastische Verhalten endet mit der Berührung der Enden der Begrenzungsstege 6, durch die ein weiterer Verformungsweg des Spannelements 1 blockiert wird.

Die Begrenzung des Verformungswegs bietet eine Schutzfunktion vor dauerhaften Verformungen. Zudem lässt sich bei einem Zusammendrücken des Spannelements 1 mit Hilfe der Begrenzungsstege 6 eine definierte Vorspannung erzielen. Diese Eigenschaft kann insbesondere beim Einsetzen des Spannelements 1 während eines operativen Eingriffs oder vorab bei einer Bemessung von Verknüpfungen mehrerer Spannelemente 1 einer Fixierungsvorrichtung vorteilhaft genutzt werden.

Die Fig. 14 zeigt eine Ausführungsform der erfindungsgemäßen Fixierungsvorrichtung, bei der zwischen den Befestigungsmitteln 2 und den Spannelementen 1 Hülsenelemente 26 aus einem resorbierbaren Kunststoff angeordnet sind. Wenn die Hülsenelemente 26 durch das umgebende Körpergewebe resorbiert werden, entstehen den Hülsenelementen 26 entsprechende Leerräume. Unter der Zugspannung des Spannelements 1 verkürzt sich der effektive Abstand zwischen den Aufnahmeabschnitten 12. Infolgedessen wird die Vorspannung des elastischen Spannelements 1 aufgehoben, sodass eine Kraftentkopplung zwischen den Befestigungsmitteln 2 stattfindet. Die Kraftübertragung der Fixierungsvorrichtung geht daher mit fortschreitender Resorption der Hülsenelemente 26 verloren, wodurch die Belastung auf den Kochen wieder ansteigt.

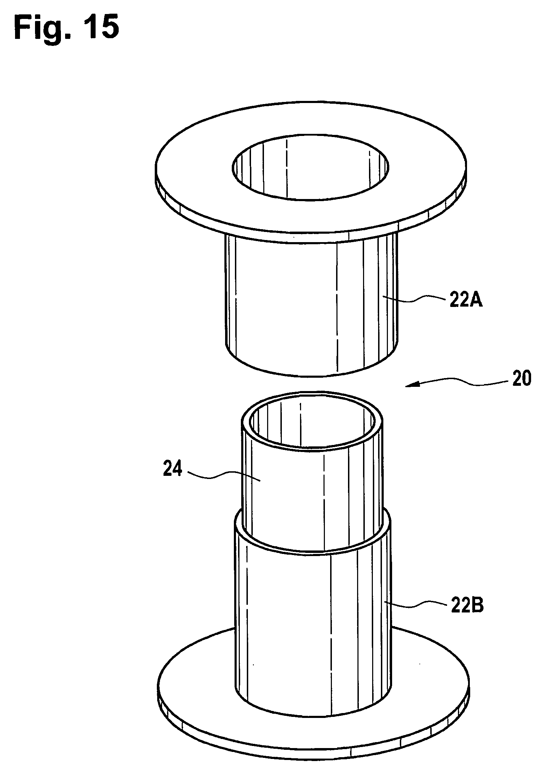

Die Spannhülse 20 in Fig. 15 umfasst einen radial inneren Abschnitt 24, in dem eine Corticalisschraube 21 aufnehmbar ist. Der radial innere Abschnitt 24 wird durch eine Passung, vorzugsweise eine Presspassung, in den radial äußeren Abschnitt 22 der Spannhülse 20 eingefügt. Der radial äußere Abschnitt 22 kann wiederum in axialer Richtung zwei Hälften 22A und 22B geteilt sein, die durch eine Passung zusammenfügbar sind. Der radial innere Abschnitt 24 kann aus einem resorbierbaren Kunststoff und der radial äußere Abschnitt 22 kann aus einem Metall ausgebildet sein, oder umgekehrt.

Wenn z.B. der radial innere Abschnitt 24 resorbiert wird, entsteht in der Spannhülse 20 eine Maßtoleranz gegenüber der Corticalisschraube 21. Wie obenstehend beschrieben, verkürzt sich somit der effektive Abstand zwischen den Aufnahmeabschnitten 12 des vorgespannten Spannelements 1. Die Vorspannung des Spannelements 1 hebt sich auf und es findet eine Kraftentkopplung der Fixierungsvorrichtung statt. Der Knochen wird somit wieder zunehmender Belastung ausgesetzt.

Die Spannhülse 20 oder einer der radial abgeteilten Abschnitte 22 und 24 desselben kann durch ein spanendes Verfahren wie z.B. durch Drehspanen aus einem der obenstehenden Metalle hergestellt werden. Das Hülsenelement 26 oder der andere der beiden Abschnitte 22 und 24 kann durch ein Gießverfahren aus einem resorbierbaren Kunststoff gegossen werden. Die radial geteilten Abschnitte 22 und 24 können miteinander vergossen oder ineinander verpresst werden. Die Spannhülse 20 oder der radial innere Abschnitt 24 weist zudem eine Durchgangsbohrung mit einem Innendurchmesser auf, der basierend auf einem Durchmesser einer Corticalisschraube 21 bestimmt wird.

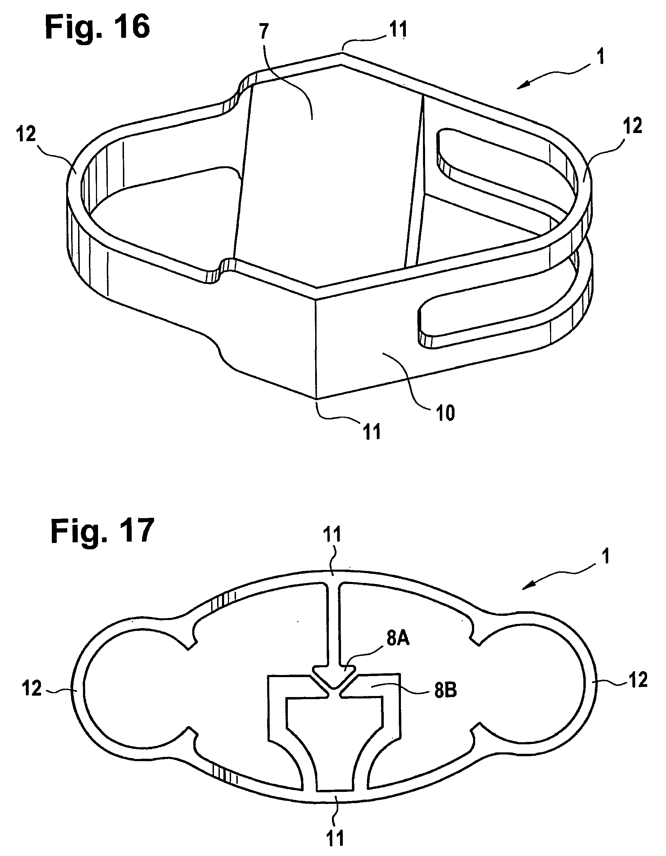

Weiterhin ist Fig. 16 ein Spannelement 1 einer resorptionsaktiven Fixierungsvorrichtung zu entnehmen, das im Laufe der Zeit seine Vorspannung verliert. Hierzu ist ein Inlay 7 aus einem resorbierbaren Kunststoff vorgesehen. Das Inlay 7 wird zwischen den Wandungen der Beugeabschnitte 11 eingespreizt, nachdem das Spannelement 1 die Befestigungsmittel 2 (nicht dargestellt) umschließt. Da die Beugeabschnitte 11 zur Erzeugung der Vorspannung bei dieser Variante nicht nach innen sonder nach außen gedrückt werden, verkürzt sich in diesem Fall sinngemäß der Abstand der Aufnahmeabschnitte 12. Somit lässt sich durch das Einspannen eines Inlays 7 mit einer vorbestimmten Länge eine definierte Zugkraft zwischen den Befestigungsmitteln 2 erzeugen. Wenn das Inlay 7 im Laufe des Heilungsprozesses resorbiert, also u.a. kürzer wird, kehren die Beugeabschnitte 11 des Spannelements 1 in den ursprünglichen entspannten Zustand zurück. Infolgedessen vergrößert sich der effektive Abstand zwischen den Aufnahmeabschnitten 12 und die Zugkraft zwischen den Befestigungsmitteln 2 wird aufgehoben.

Ferner ist in Fig. 17 ein Spannelement 1 abgebildet, dass in einer Lage mit einer vordefinierten Vorspannung verrastet werden kann, wenn die Beugeabschnitte 11 aufeinander zu gedrückt werden. Dabei rastet z.B. ein pfeilförmiges Element 8A mittels eines Hinterschnitts in einem Fallenelement 8B ein. Bei Auswahl einer geringen Höhe der Rastelemente 8, kann die Rastverbindung z.B. durch ein entgegengesetztes Verschieben der Rastelemente in Höhenrichtung auf einfache Weise gelöst werden. Hierdurch kann die Bemaßung der Abstände der Befestigungsmittel 2 sowie die Befestigung der Fixierungsvorrichtung erleichtert werden.

Die Erfindung lässt neben den gezeigten Ausführungsformen weitere Gestaltungsansätze zu.

So kann für den operativen Eingriff zur Befestigung der Fixierungsvorrichtung beispielsweise ein Werkzeug mit verschiedenen Fassungen bereitgestellt werden, in die ein Spannelement 1 zunächst eingesetzt wird. Anhand einer festgelegten Lage der Seitenflanken wird dass Spannelement 1 in einer definierten Vorspannung gehalten. Nachdem die Befestigungsmittel 2 durch das Spannelement 1 hindurchgesteckt und in die Bruchenden des Knochens eingebracht sind, kann dann die Fassung des Werkzeugs abgezogen werden, wodurch eine vorbestimmte Zugkraft auf die Befestigungsmittel 2 wirkt. Derartige Fassungen können auch für eine Anordnung mehrerer Spannelemente 1 individuell kombinierbar vorgesehen werden, um einen operativen Eingriffs zeitsparend vorzubereiten.

Ferner ist es auch möglich das Spannelement 1 mit einem Gerippe oder Stegen konstruktiv zu verstärken, um eine höhere Festigkeit des Spannelements 1 in einer gewünschten Belastungsrichtung zu erlangen.

Überdies ist es auch möglich den Wandungsquerschnitt des Spannelements 1 in bestimmten Abschnitten zu variieren, um ein lokales Verhältnis zwischen Elastizität und Festigkeit des Spannelements 1 zu beeinflussen.

Mit der vorstehend diskutierten Erfindung wird eine Fixierungsvorrichtung zum Fixieren einer Knochenfraktur bereitgestellt, die Spannelemente umfasst, mit einem im Querschnitt im Wesentlichen hohlen, von einer umlaufenden Wandung, insbesondere mit im Wesentlichen gleichbleibender Wandstärke, umfassten, geschlossenen Konturkörper aufweist. Das Spannelement weist zudem zwei gegenüberliegende stirnseitige Aufnahmeabschnitte zur Aufnahme von Befestigungsmitteln, vorzugsweise Corticalisschrauben, die durch den Konturkörper hindurchsteckbar sind, auf. Jedes Spannelement weist weiterhin zwei Seitenflanken mit Beugeabschnitten auf, wobei die umlaufende Wandung des Konturkörpers wenigstens abschnittsweise, insbesondere im Bereich der stirnseitigen Aufnahmeabschnitte und im Bereich der seitlichen Beugeabschnitte, federelastisch verformbar ist. Dabei ist über die Beugeabschnitte ein definiertes elastisches Verhalten des Spannelements derart einprägbar, dass eine vorbestimmte Spannung zwischen den Aufnahmeabschnitten erzeugbar ist. Weiterhin ist erfindungsgemäß ein Verfahren zur Herstellung des Spannelements und eine Fixierungsvorrichtung zur Anwendung des Spannelements vorgesehen.

## Patentansprüche

1. Fixierungsvorrichtung für Knochenfrakturen, aufweisend eine Mehrzahl von Spannelementen (1) und wenigstens zwei Befestigungsmittel (2), vorzugsweise Corticalisschrauben, die im Bereich der Bruchenden in den Knochen einbringbar sind, wobei

   durch eine Kombination mehrerer Spannelemente (1) diese zu einer Kette oder sternförmig oder in einer beliebigen Kombination aus Ketten- und Sternanordnungen angeordnet werden können;

   jedes Spannelement (1) aus der Mehrzahl von Spannelementen (1) einen im Querschnitt im Wesentlichen hohlen, von einer umlaufenden Wandung (10), insbesondere mit im Wesentlichen gleichbleibender Wandstärke, umfassten, geschlossenen Konturkörper aufweist, mit:

      zwei gegenüberliegenden stirnseitigen Aufnahmeabschnitten (12) zur Aufnahme der Befestigungsmittel (2), und
      zwei Seitenflanken mit Beugeabschnitten (11), wobei

   die umlaufende Wandung (10) des Konturkörpers wenigstens abschnittsweise im Bereich der stirnseitigen Aufnahmeabschnitte (12) und im Bereich der seitlichen Beugeabschnitte (11), federelastisch verformbar ist,
   über die Beugeabschnitte (11) ein definiertes elastisches Verhalten des Spannelements (1) derart eingeprägt ist, dass eine vorbestimmte Spannung zwischen den Aufnahmeabschnitten (12) erzeugbar ist,
   die Befestigungsmittel (2) durch den Konturkörper hindurchsteckbar sind,
   **dadurch gekennzeichnet, dass**
   die stirnseitigen Aufnahmeabschnitte (12) derart ausgebildet sind, dass sie die Befestigungsmittel (2) nicht umschließen, wenigstens eine Spannhülse (20) und/oder ein Hülsenelement (26) vorgesehen ist in welcher/welchem jeweils ein Befestigungsmittel (2) der zumindest zwei Befestigungsmittel (2) aufnehmbar ist, und
   dass sich miteinander kombinierte, benachbarte Spannelemente (1) derart überschneiden, dass das Befestigungsmittel (2), die Spannhülse (20) oder das Hülsenelement (26) von den jeweiligen stirnseitigen Aufnahmeabschnitten (12) der überschneidenden Spannelemente (1) umschlossen wird.

2. Fixierungsvorrichtung nach Anspruch 1, wobei die Spannhülse (20) vorzugsweise zwei stirnseitige Kröpfungen umfasst, die im Wesentlichen entsprechend einer Wandungshöhe eines Spannelements (1) voneinander beabstandet sind.

3. Fixierungsvorrichtung nach Anspruch 2, wobei die Spannhülse (20) zweiteilig ausgebildet ist und in axialer Richtung zusammenfügbar ist, sodass die Wandung (10) des Spannelements (1) zwischen den Kröpfungen einspannbar ist.

4. Fixierungsvorrichtung nach Anspruch 2 oder 3, wobei die Spannhülse (20) radial in zwei Abschnitte (22, 24) aufgeteilt ist, und der radial innere Abschnitt (24) oder der radial äußere Abschnitt (22) aus einem resorbierbaren Kunststoff ausgebildet ist.

5. Fixierungsvorrichtung nach einem der Ansprüche 1 bis 4, wobei das Hülsenelement (26) aus einem resorbierbaren Kunststoff ausgebildet ist.

6. Fixierungsvorrichtung nach einem der Ansprüche 1 bis 5, wobei zwischen den Beugeabschnitten (11) wenigstens eines Spannelements (1) ein Inlay (7) eingespreizt ist, dessen Länge die Beugeabschnitte (11) gegen eine Vorspannung des Spannelements (1) auseinander drückt, wobei das Inlay (7) aus einem resorbierbaren Material besteht.

7. Fixierungsvorrichtung nach einem der Ansprüche 1 bis 6, wobei ein Innenradius der stirnseitigen Aufnahmeabschnitte (12) dem Außenradius eines Umfangsflächenabschnitts des Befestigungsmittels (2), des Hülsenelements (26) oder der Spannhülse (20) entspricht.

8. Fixierungsvorrichtung nach Anspruch 1, wobei der geschlossene Konturkörper des Spannelements (1) im Wesentlichen oval, rautenförmig, doppel-rautenförmig oder brillenförmig ausgebildet ist.

9. Fixierungsvorrichtung nach Anspruch 1, wobei der geschlossene Konturkörper des Spannelements (1) eine Krümmung in Bezug auf die Erstreckungsebene der Kontur aufweist.

10. Fixierungsvorrichtung nach Anspruch 1, wobei die Wandung (10) im Bereich der Aufnahmeabschnitte (12) Durchbrüche (14) und/oder Ausschnitte (13) aufweist, die derart ausgebildet sind, dass durch eine Nut-Gestalt und/oder eine Feder-Gestalt von Wandungsabschnitten eine komplementäre Passung bereitgestellt ist, vermittels der sich die Konturen von benachbarten Spannelementen (1) in Überschneidung bringen lassen.

11. Fixierungsvorrichtung nach Anspruch 1, wobei an der Außenfläche der Wandung (10) Kupplungselemente (3A, 3B) oder ein Kugelelement (4A) oder ein sphärisches Klemmelement (4B) vorgesehen sind, vermittels denen sich benachbarte Spannelemente (1), vorzugsweise kugelgelenkig gegeneinander drehbar und/oder zueinander lagefixiert, verbinden lassen.

12. Fixierungsvorrichtung nach Anspruch 1, wobei an der Wandung (10) Befestigungsaugen (5) vorgesehen sind, durch welche die Befestigungsmittel (2) hindurchsteckbar sind.

13. Fixierungsvorrichtung nach Anspruch 1, wobei an zwei gegenüberliegenden Wandungsabschnitten aufeinander zuweisende Begrenzungsstege (6) ausgebildet sind, deren Enden sich bei einer Verformung des Spannelements (1) berühren.

14. Fixierungsvorrichtung nach Anspruch 1, wobei innerhalb des Konturkörpers des Spannelements (1) an den gegenüberliegenden Beugeabschnitten (11) zwei aufeinander zu gerichtete, miteinander in Eingriff bringbare Rastelemente (8), vorzugsweise ein hinterschnittenes Element (8A) und ein Fallenelement (8B), angeordnet sind, vermittels denen die Beugeabschnitte (11) in einer vorgespannten Lage des Spannelements (1) rastend, wieder lösbar, verbindbar sind.

15. Verfahren zum Herstellen einer Fixierungsvorrichtung für Knochenfrakturen nach einem der Ansprüche 1 bis 14, zum Fixieren einer Knochenfraktur, mit den Schritten:

Auswählen eines stranggepressten Hohlprofils;
Abtrennen einer Mehrzahl von Spannelemente (1) bildenden Hohlprofilabschnitten an einem in Längsrichtung des Hohlprofils vorbestimmten Querschnitt, der die Höhe eines jeden Spannelements (1) bildet;
Abtragen von Ausschnitten (13) oder Durchbrüchen (14) in der Wandung (10) des Hohlprofilabschnitts, zur Ausbildung eines im Querschnitt im Wesentlichen hohlen, von einer umlaufenden Wandung (10) umfassten, geschlossenen Konturkörpers; und
Kombinieren mehrerer Spannelemente (1) zu einer Kette oder sternförmig oder in einer beliebigen Kombination aus Ketten- und Sternanordnungen unter Verwendung von zumindest zwei Befestigungsmitteln (2), die durch den Konturkörper hindurchsteckbar sind, einer Spannhülse (20) und/oder einem Hülsenelement (26), in welcher/welchem jeweils ein Befestigungsmittel (2) der zumindest zwei Befestigungsmittel (2) aufnehmbar ist;

wobei sich miteinander kombinierte, benachbarte Spannelemente (1) derart überschneiden, dass das Befestigungsmittel (2), die Spannhülse (20) oder das Hülsenelement (26) von jeweilis in den Spannelementen (1) ausgebildeten, stirnseitigen Aufnahmeabschnitten (12) sich überschneidender Spannelemente (1) umschlossen wird.

**16.** Verfahren zum Herstellen einer Fixierungsvorrichtung für Knochenfrakturen nach einem der Ansprüche 1 bis 14, mit den Schritten:

Auswählen eines stranggepressten Vollprofils;

Abtrennen einer Mehrzahl von Spannelemente (1) bildenden Vollprofilabschnitten an einem in Längsrichtung des Vollprofils vorbestimmten Querschnitt, der die Höhe eines jeden Spannelements (1) bildet;

Spanabhebendes Bearbeiten des Vollmaterials zur Ausbildung eines im Querschnitt im Wesentlichen hohlen, von einer umlaufenden Wandung (10) umfassten, geschlossenen Konturkörpers, insbesondere mit im Wesentlichen gleichbleibender Wandstärke;

Abtragen von Ausschnitten (13) oder Durchbrüchen (14) in der Wandung (10) des geschlossenen Konturkörpers; und

Kombinieren mehrerer Spannelemente (1) zu einer Kette oder sternförmig oder in einer beliebigen Kombination aus Ketten- und Sternanordnungen unter Verwendung von zumindest zwei Befestigungsmitteln (2), die durch den Konturkörper hindurchsteckbar sind, einer Spannhülse (20) und/oder einem Hülsenelement (26), in welcher/welchem jeweils ein Befestigungsmittel (2) der zumindest zwei Befestigungsmittel (2) aufnehmbar ist;

wobei sich miteinander kombinierte, benachbarte Spannelemente (1) derart überschneiden, dass das Befestigungsmittel (2), die Spannhülse (20) oder das Hülsenelement (26) von jeweils in den Spannelementen (1) ausgebildeten, stirnseitigen Aufnahmeabschnitten (12) sich überschneidender Spannelemente (1) umschlossen wird.

**Claims**

**1.** A fixation device for bone fractures, comprising a plurality of clamping elements (1) and at least two fixing means (2), preferably cortical screws, which can be inserted into the bone in the region of the ends of a fracture, wherein by a combination of several clamping elements (1) said clamping elements (1) can be arranged to form a chain or in a star-shaped manner or in any other combination of chain-shaped and star-shaped arrangements; each clamping element (1) from the plurality of clamping elements (1) comprises a closed contoured body which has a substantially hollow cross-section and is enclosed by a peripheral wall (10), which is in particular of a substantially constant thickness, comprising:

two opposite receiving portions (12) at end faces of said contoured body, said receiving portions (12) being intended for receiving fixing means (2), and
two lateral flanks having angled portions (11), wherein

the peripheral wall (10) of the contoured body is resiliently deformable at least in sections in the region of the receiving portions (12) on the end faces of the same and in the region of the lateral angled portions (11),
a defined resilient behaviour of the clamping element (1) is impressed via the angled portions (11) in such a way that a predetermined tension can be generated between the receiving portions (12),
the fixing means (2) can be pushed through the contoured body,
**characterized in that**
the receiving portions (12) on the end faces are formed such that they do not enclose the fixing means (2),
at least one clamping sleeve (20) and/or one sleeve element (26) is provided, in which one fixing means (2), respectively, of the at least two fixing means (2) can be received, and
that adjacent clamping elements (1), which are combined with one another, overlap each other in such a manner that the fixing means (2), the clamping sleeve (20) or the sleeve element (26) is enclosed by the respective receiving portions (12) on the end faces of the overlapping clamping elements (1).

**2.** The fixation device according to claim 1, wherein the clamping sleeve (20) preferably comprises two cranks on the end faces thereof, which are spaced apart from each other substantially in correspondence with a wall height of a clamping element (1).

**3.** The fixation device according to claim 2, wherein the clamping sleeve (20) is formed in a two-part manner and can be joined together in an axial direction so that the wall (10) of the clamping element (1) can be wedged in between the cranks.

**4.** The fixation device according to claim 2 or 3, wherein the clamping sleeve (20) is radially divided into two sections (22, 24), and the radially inner section (24) or the radially outer section (22) is made of an absorbable material.

5. The fixation device according to anyone of claims 1 through 4, wherein the sleeve element (26) is made of an absorbable synthetic material.

6. The fixation device according to anyone of claims 1 through 5, wherein between the angled portions (11) of at least one clamping element (1) an inlay (7) is wedged, the length of which presses apart the angled portions (11) against a pretension of the clamping element (1), wherein the inlay (7) consists of an absorbable material.

7. The fixation device according to anyone of claims 1 through 6, wherein an inner radius of the receiving portions (12) at the end faces corresponds to the outer radius of a peripheral area section of the fixing means (2), of the sleeve element (26) or of the clamping sleeve (20).

8. The fixation device according to claim 1, wherein the closed contoured body of the clamping element (1) is substantially of an oval, rhomboidal, double-rhomboidal or spectacles-shaped form.

9. The fixation device according to claim 1, wherein the closed contoured body of the clamping element (1) has a curvature with respect to the plane of extension of the contour.

10. The fixation device according to claim 1, wherein, in the region of the receiving portions (12), the wall (10) comprises openings (14) and/or cutaway portions (13) which are formed such that by a groove structure and/or a tongue structure of wall sections a complementary fit is provided by means of which the contours of adjacent clamping elements (1) can be caused to overlap each other.

11. The fixation device according to claim 1, wherein at the outer surface of the wall (10) coupling elements (3A, 3B) or a ball element (4A) or a spherical clamping part (4B) is provided by means of which adjacent clamping elements (1) can be connected with each other preferably in such a manner that they are rotatable with respect to each other via a ball joint and/or that they are fixed in their position with respect to each other.

12. The fixation device according to claim 1, wherein at the wall (10) there are provided fastening eyes (5) through which the fixing means (2) can be pushed.

13. The fixation device according to claim 1, wherein at two opposing wall sections there are formed limiting bars (6) pointing towards each other, the ends of which come into contact with each other when the clamping element (1) is deformed.

14. The fixation device according to claim 1, wherein within the contoured body of the clamping element (1) at the opposite angled portions (11) there are arranged two snap-in locking elements (8) which are pointing towards each other and which can be brought into engagement with each other, preferably an undercut element (8A) and a catch element (8B), by means of which the angled portions (11) can be connected in a pretensioned position of the clamping element (1) in a locking and again releasable manner.

15. A method for producing a fixation device for bone fractures according to anyone of claims 1 through 14 for setting a bone fracture, comprising the following steps:

> selecting an extruded hollow profile;
> cutting off a plurality of hollow-profile sections which form clamping elements (1) at a cross-section predetermined in the longitudinal direction of the hollow profile and forming the height of the clamping element (1),
> cutting cutaway portions (13) or openings (14) into the wall (10) of the hollow-profile section to thereby form a closed contoured body which has a substantially hollow cross-section and is enclosed by a peripheral wall (10); and
> combining several clamping elements (1) to form a chain or in a star-shaped manner or in any other combination of chain-shaped and star-shaped arrangements using at least two fixing means (2) that can be pushed through the contoured body, one clamping sleeve (20) and/or one sleeve element (26) in which one fixing means (2), respectively, of the at least two fixing means (2) can be received;

wherein adjacent clamping elements (1) which are combined with one another overlap each other in such a manner that the fixing means (2), the clamping sleeve (20) or the sleeve element (26) is enclosed by the respective receiving portions (12), formed at the end faces of the clamping elements (1), of the overlapping clamping elements (1).

16. A method for producing a fixation device for bone fractures according to anyone of claims 1 through 14 for setting a bone fracture, comprising the following steps:

> selecting an extruded solid profile;
> cutting off a plurality of solid-profile sections which form clamping elements (1) at a cross-section predetermined in the longitudinal direction of the solid profile and forming the height of the clamping element (1),
> machining the solid material for forming a closed contoured body which has a substantially hollow cross-section and is enclosed by a peripheral wall (10) which is in particular of a substantially constant thickness;
> cutting cutaway portions (13) or openings (14) into the wall (10) of the closed contoured body; and
> combining several clamping elements (1) to form a chain or in a star-shaped manner or in any other combination of chain-shaped and star-shaped arrangements using at least two fixing means (2) that can be pushed through the contoured body, one clamping sleeve (20) and/or one sleeve element (26) in which one fixing means (2), respectively, of the at least two fixing means (2) can be received;

> wherein adjacent clamping elements (1) which are combined with one another overlap each other in such a manner that the fixing means (2), the clamping sleeve (20) or the sleeve element (26) is enclosed by the respective receiving portions (12), formed at the end faces of the clamping elements (1), of the overlapping clamping elements (1).

**Revendications**

1. Dispositif de fixation pour des fractures osseuses, présentant une pluralité d'éléments de serrage (1) et au moins deux moyens de fixation (2), de préférence des vis corticales qui peuvent être introduites dans la zone des extrémités fracturées dans les os,

   par une combinaison de plusieurs éléments de serrage (1), ceux-ci pouvant être agencés en une chaîne ou en forme d'étoile ou dans une combinaison quelconque d'agencements de chaîne et étoile ;
   chaque élément de serrage (1) de la pluralité d'éléments de serrage (1) présentant un corps de contour fermé, compris par une paroi tournante (10) en particulier avec une épaisseur de paroi sensiblement constante, sensiblement creux en section transversale, avec :

   > deux sections de réception (12) côté avant en regard pour la réception des moyens de fixation (2) et
   > deux flancs latéraux avec des sections de pliage (11),
   > la paroi tournante (10) du corps de contour étant déformable élastiquement au moins par sections dans la zone des sections de réception (12) côté avant et dans la zone des sections de pliage (11) latérales,
   > un comportement élastique défini de l'élément de serrage (1) étant appliqué par les sections de pliage (11) de telle manière qu'une tension prédéterminée puisse être générée entre les sections de réception (12),
   > les moyens de fixation (2) étant enfichables par le corps de contour,

   **caractérisé en ce que**

   > les sections de réception (12) côté avant sont réalisées de telle manière qu'elles n'entourent pas les moyens de fixation (2),
   > au moins une douille de serrage (20) et/ou un élément de douille (26) est prévu, dans laquelle/lequel respectivement un moyen de fixation (2) d'au moins deux moyens de fixation (2) peut être reçu et

   **en ce que**

   > des éléments de serrage (1) contigus, combinés entre eux se coupent de telle manière que le moyen de fixation (2), la douille de serrage (20) ou l'élément de douille (26) soit entouré par les sections de réception (12) côté avant respectives des éléments de serrage (1) se coupant.

2. Dispositif de fixation selon la revendication 1, la douille de serrage (20) comportant de préférence deux coudes côté avant qui sont espacés l'un de l'autre sensiblement selon une hauteur de paroi d'un élément de serrage (1).

3. Dispositif de fixation selon la revendication 2, la douille de serrage (20) étant réalisée en deux parties et étant assemblable dans le sens axial de sorte que la paroi (10) de l'élément de serrage (1) puisse être serrée entre les coudes.

**4.** Dispositif de fixation selon la revendication 2 ou 3, la douille de serrage (20) étant divisée radialement en deux sections (22, 24) et la section radialement intérieure (24) ou la section radialement extérieure (22) étant réalisée en un plastique résorbable.

**5.** Dispositif de fixation selon l'une quelconque des revendications 1 à 4, l'élément de douille (26) étant réalisé en un plastique résorbable.

**6.** Dispositif de fixation selon l'une quelconque des revendications 1 à 5, un inlay (7) étant écarté entre les sections de pliage (11) d'au moins un élément de serrage (1), dont la longueur écarte les sections de pliage (11) contre une précontrainte de l'élément de serrage (1) les unes des autres, l'inlay (7) se composant d'un matériau résorbable.

**7.** Dispositif de fixation selon l'une quelconque des revendications 1 à 6, un rayon intérieur des sections de réception côté avant (12) correspondant au rayon extérieur d'une section de surface périphérique du moyen de fixation (2), de l'élément de douille (26) ou de la douille de serrage (20).

**8.** Dispositif de fixation selon la revendication 1, le corps de contour fermé de l'élément de serrage (1) étant réalisé sensiblement ovale, en forme de losange, en forme de double losange ou de lunette.

**9.** Dispositif de fixation selon la revendication 1, le corps de contour fermé de l'élément de serrage (1) présentant une courbure en référence au plan d'étirement du contour.

**10.** Dispositif de fixation selon la revendication 1, la paroi (10) dans la zone des sections de réception (12) présentant des interruptions (14) et/ou des découpes (13) qui sont réalisées de telle manière qu'une adaptation complémentaire soit mise à disposition par une forme de rainure et/ou une forme de ressort des sections de paroi, à l'aide de laquelle les contours d'éléments de serrage (1) contigus peuvent être amenés en chevauchement.

**11.** Dispositif de fixation selon la revendication 1, des éléments de couplage (3A, 3B) ou un élément sphérique (4A) ou un élément de serrage (4B) sphérique étant prévu sur la surface extérieure de la paroi (10), à l'aide desquels des éléments de serrage (1) contigus peuvent être reliés de préférence par articulation sphérique, de manière rotative l'un contre l'autre et/ou tout en étant fixés en position l'un par rapport à l'autre.

**12.** Dispositif de fixation selon la revendication 1, des oeillets de fixation (5) étant prévus sur la paroi (10), par lesquels les moyens de fixation (2) sont enfichables.

**13.** Dispositif de fixation selon la revendication 1, des nervures de limitation (6) tournées l'une vers l'autre étant réalisées sur deux sections de paroi en regard, dont les extrémités se touchent lors d'une déformation de l'élément de serrage (1).

**14.** Dispositif de fixation selon la revendication 1, deux éléments d'encliquetage (8) pouvant être amenés en engagement l'un avec l'autre, dirigés l'un vers l'autre, de préférence un élément contre-dépouillé (8A) et un élément à loquet (8B) étant agencés dans le corps de contour de l'élément de serrage (1) sur les sections de pliage (11) en regard, à l'aide desquels les sections de pliage (11) peuvent être reliées par encliquetage, de nouveau de manière détachable, dans une position précontrainte de l'élément de serrage (1).

**15.** Procédé de fabrication d'un dispositif de fixation pour des fractures osseuses selon l'une quelconque des revendications 1 à 14, pour la fixation d'une fracture osseuse, avec les étapes suivantes :

la sélection d'un profil creux extrudé ;
la séparation d'une pluralité de sections de profil creux formant des éléments de serrage (1) sur une section transversale prédéterminée dans le sens longitudinal du profil creux qui forme la hauteur de chaque élément de serrage (1) ;
l'enlèvement de découpes (13) ou d'interruptions (14) dans la paroi (10) de la section de profil creux pour la réalisation d'un corps de contour fermé compris par une paroi tournante (10), creux sensiblement en section transversale ; et
la combinaison de plusieurs éléments de serrage (1) en une chaîne ou en forme d'étoile ou en une combinaison quelconque d'agencements de chaîne et d'étoile en utilisant au moins deux moyens de fixation (2) qui sont enfichables par le corps de contour, d'une douille de serrage (20) et/ou d'un élément de douille (26), dans laquelle/lequel respectivement un moyen de fixation (2) des au moins deux moyens de fixation (2) peut être reçu ;

des éléments de serrage (1) contigus combinés entre eux se coupant de telle manière que le moyen de fixation (2), la douille de serrage (20) ou l'élément de douille (26) soit entouré par respectivement des sections de réception (12) côté avant, réalisées dans les éléments de serrage (1), des éléments de serrage (1) se coupant.

16. Procédé de fabrication d'un dispositif de fixation pour des fractures osseuses selon l'une quelconque des revendications 1 à 14, avec les étapes suivantes :

la sélection d'un profil plein extrudé;

la séparation d'une pluralité de sections de profil plein formant des éléments de serrage (1) sur une section transversale prédéterminée dans le sens longitudinal du profil plein, laquelle forme la hauteur de chaque élément de serrage (1) ;

l'usinage par enlèvement de copeaux du matériau plein pour la réalisation d'un corps de contour fermé, compris par une paroi tournante (10), sensiblement creux en section transversale, en particulier avec une épaisseur de paroi sensiblement constante ;

l'enlèvement de découpes (13) ou d'interruptions (14) dans la paroi (10) du corps de contour fermé ; et

la combinaison de plusieurs éléments de serrage (1) en une chaîne ou une forme d'étoile ou en une combinaison quelconque d'agencements de chaîne et d'étoile en utilisant au moins deux moyens de fixation (2) qui peuvent être enfichés par le corps de contour, d'une douille de serrage (20) et/ou d'un élément de douille (26), dans laquelle/lequel respectivement un moyen de fixation (2) des au moins deux moyens de fixation (2) peut être reçu ;

des éléments de serrage (1) contigus, combinés entre eux se coupant de telle manière que le moyen de fixation (2), la douille de serrage (20) ou l'élément de douille (26) soit entouré respectivement par des sections de réception (12) côté avant, réalisées dans les éléments de serrage (1), d'éléments de serrage se coupant (1).

**Fig. 1**

**Fig. 2**

**Fig. 3**

20A

1

20B

**Fig. 4**

1

13

12

11

12

14

13

13

11

10

**Fig. 5**

11
13
12
11 1 11
12
14
13
13
11 11
10
11

**Fig. 6**

11
1
12
14
13
12
13
11 10

# Fig. 7

# Fig. 8

**Fig. 9**

**Fig. 10A**

**Fig. 10B**

**Fig. 10C**

## Fig. 11A

4A

1

4B

## Fig. 11B

4B

4A

4C

4A

1

4B

4A

1

## Fig. 11C

4C

4B

1

4A

1

## Fig. 12A

5

5

3A

3A

3B

3B

1

1

5

5

5

## Fig. 12B

21

5

5

21

1

**Fig. 13**

6

6

1

**Fig. 14**

21

26

26

26

1A

1B

1C

# Fig. 15

**Fig. 16**

**Fig. 17**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7763056 B2
- DE 20001879 U1
- WO 2004034916 A1
- WO 2005086708 A2
- WO 2006031692 A2